# EUROPEAN PATENT SPECIFICATION

(11) **EP 4 478 963 B1**
(45) Date of publication and mention of the grant of the patent: **14.10.2026**
(21) Application number: 23708331.6
(22) Date of filing: 08.02.2023
(51) Int. Cl.: A61B 17/11

(54) **SHUNT IMPLANT DEVICE WITH ADJUSTABLE BARREL**
SHUNT-IMPLANTATVORRICHTUNG MIT EINSTELLBAREM LAUF
DISPOSITIF D'IMPLANT DE DÉRIVATION À CYLINDRE RÉGLABLE

(30) Priority: 14.02.2022 US 202263309761 P
(43) Date of publication of application: 25.12.2024
(73) Proprietor: Edwards Lifesciences Corporation, Irvine, CA 92614 (US)
(72) Inventor: VALDEZ, Michael G., Irvine, CA 92614 (US); TRAN, Tiana, Irvine, CA 92614 (US)
(74) Representative: Eisenführ Speiser
(86) International application number: PCT/US2023/012577
(87) International publication number: WO 2023/154308

(56) References cited:
- WO-A1-2021/086707
- US-A1- 2008 071 178

## Description

### RELATED APPLICATION

This application claims priority to U.S. Provisional Patent Application Serial No. 63/309,761, filed on February 14, 2022 and entitled SHUNT IMPLANT DEVICE WITH ADJUSTABLE BARREL.

### BACKGROUND

The present disclosure generally relates to the field of medical implant devices. Various medical procedures involve the implantation of a medical implant devices within the anatomy of the heart. Certain physiological parameters associated with such anatomy, such as fluid pressure, can have an impact on patient health prospects.

WO 2021/086706 A1 relates to a sensor-retention structure that includes a sensor-support strut and a means for securing a sensor device to the sensor-support strut. A sensor- support arm can be configured to have disposed thereon an at least partially cylindrical sensor device and be associated with one or more sensor-retention fingers projecting from the sensor- support arm and/or a cage structure configured to be secured to the sensor device.

Further, US 2008/0071178 A1 relates to an anchor for an implantable medical device, such as an implantable physiologic sensor which includes a proximal hub portion, an intermediate portion extending radially and distally from the hub portion, and a distal portion extending distally from the proximal portion and adapted to engage an inner surface of a target vessel such as to secure the implantable medical device therein. The anchor can assume a collapsed configuration for delivery through a catheter, and an expanded configuration for fixation within the vessel once deployed. The intermediate portion extends from the proximal portion at an oblique angle, allowing the anchor to be retracted and recollapsed within the delivery catheter after initial deployment, if re-positioning or removal of the implantable medical device is necessary or desired.

### SUMMARY

The claimed invention is defined by independent claim 1. The dependent claims specify preferred embodiments of the claimed invention.

Described herein are one or more methods and/or devices to facilitate the shunting of blood between chambers and/or vessels of a patient's cardiac and/or circulatory system. Examples of the present disclosure further relate to the monitoring of physiological parameter(s) associated with certain chambers and/or vessels of the heart, such as the left atrium, using one or more shunt-type sensor implant devices.

For purposes of summarizing the disclosure, certain aspects, advantages and novel features have been described. It is to be understood that not necessarily all such advantages may be achieved in accordance with any particular example. Thus, the disclosed examples may be carried out in a manner that achieves or optimizes one advantage or group of advantages as taught herein without necessarily achieving other advantages as may be taught or suggested herein.

### BRIEF DESCRIPTION OF THE DRAWINGS

Various examples are depicted in the accompanying drawings for illustrative purposes and should in no way be interpreted as limiting the scope of the inventions. In addition, various features of different disclosed examples can be combined to form additional examples, which are part of this disclosure. Throughout the drawings, reference numbers may be reused to indicate correspondence between reference elements.
Figure 1 illustrates human cardiac anatomy in accordance with one or more examples.
Figure 2 illustrates a superior view of a human heart in accordance with one or more examples.
Figure 3A shows a perspective view of a shunt structure in accordance with one or more examples.
Figure 3B shows a side view of the shunt structure of Figure 3A, including a detailed view of a barrel portion of the shunt structure, in accordance with one or more examples.

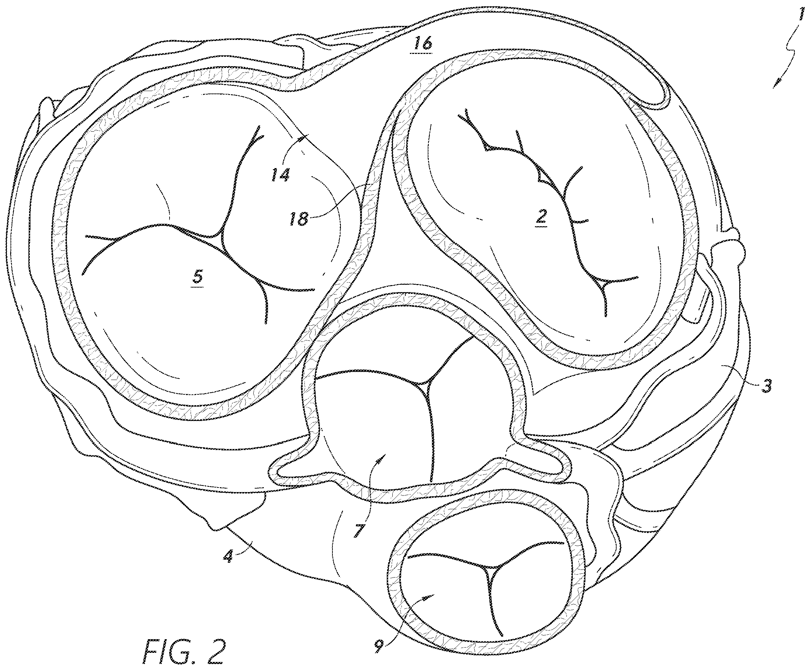

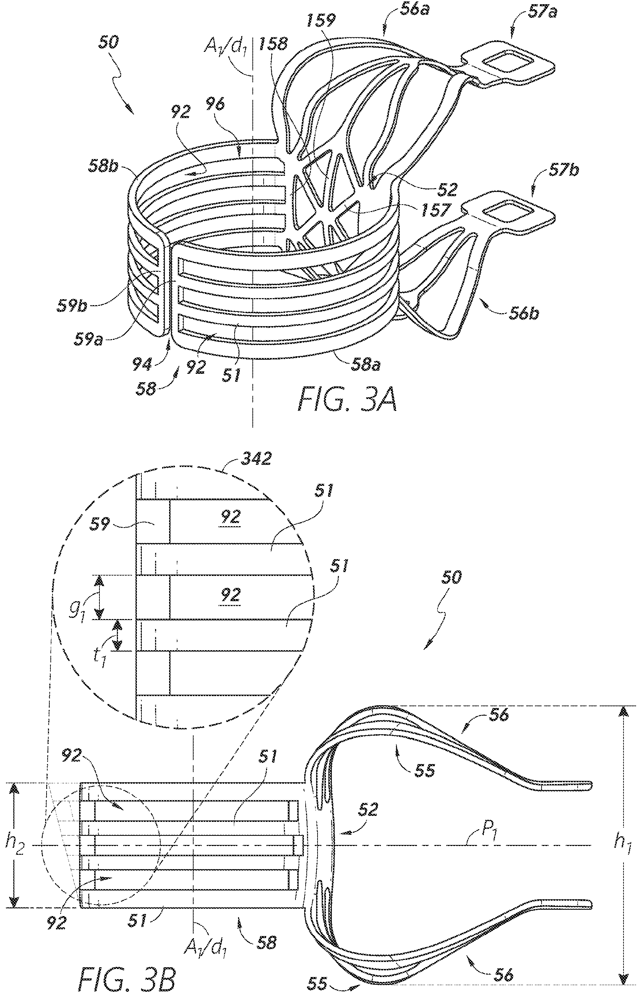

Figures 3C-1 and 3C-2 show axial views of the shunt structure of Figures 3A and 3B with the barrel thereof in expanded and compressed configurations, respectively, in accordance with one or more examples.

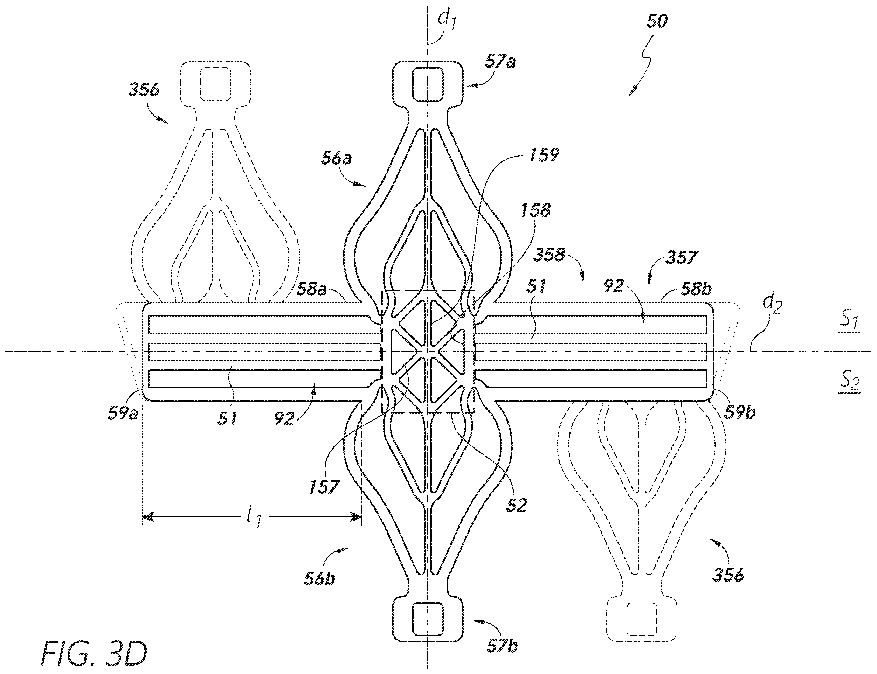

Figure 3D shows the shunt structure of Figure 3A in a flattened configuration in accordance with one or more examples.

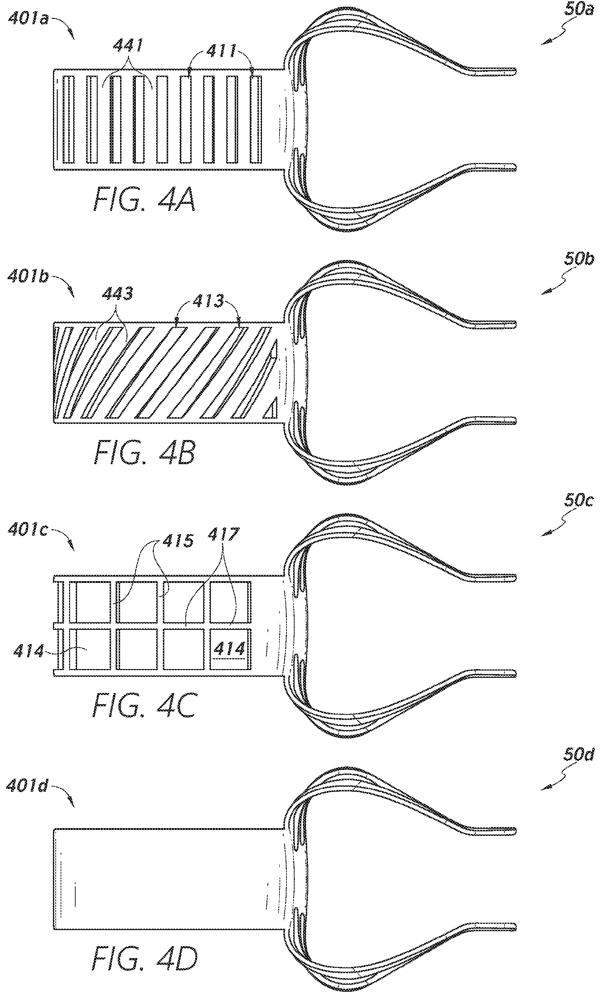

Figures 4A-4D show side views of shunt structures having various barrel designs in accordance with a plurality of examples.

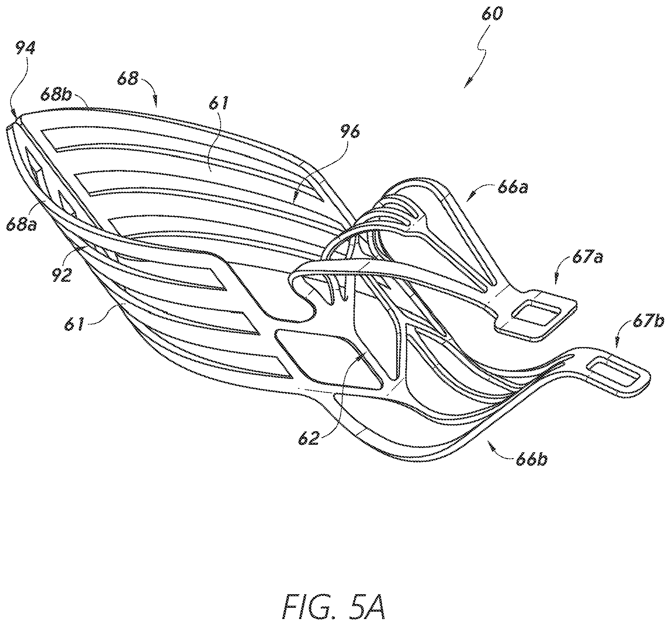

Figure 5A shows a perspective view of a shunt structure having a canted barrel in accordance with one or more examples.

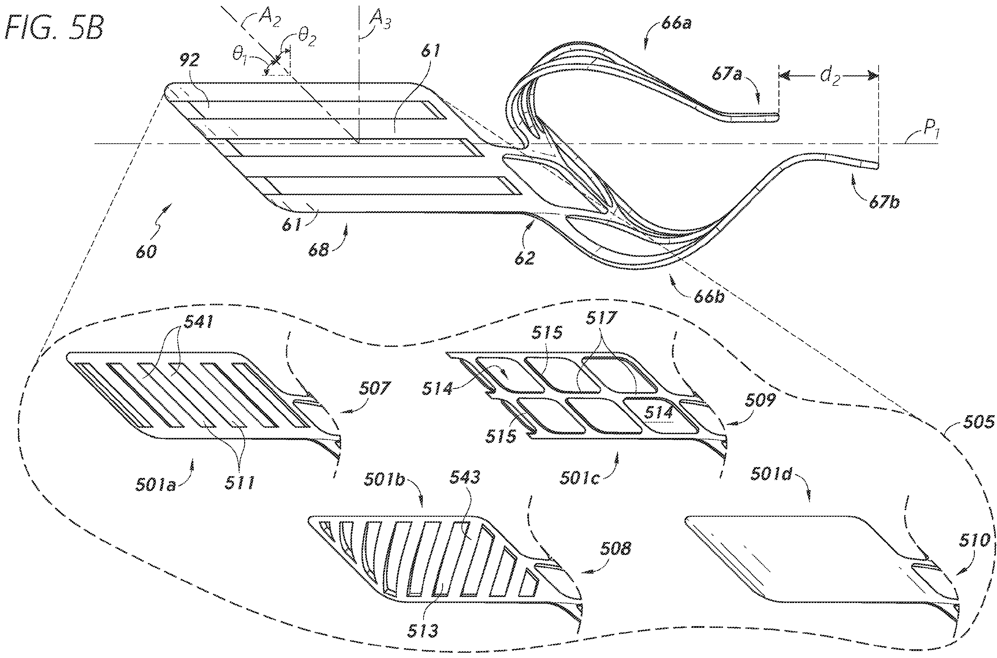

Figure 5B shows a side view of the shunt structure of Figure 5A, as well as various alternative canted barrel designs, in accordance with one or more example.
Figures 5C-1 and 5C-2 show axial views of the shunt structure of Figures 5A and 5B with the barrel thereof in expanded and compressed configurations, respectively, in accordance with one or more examples.

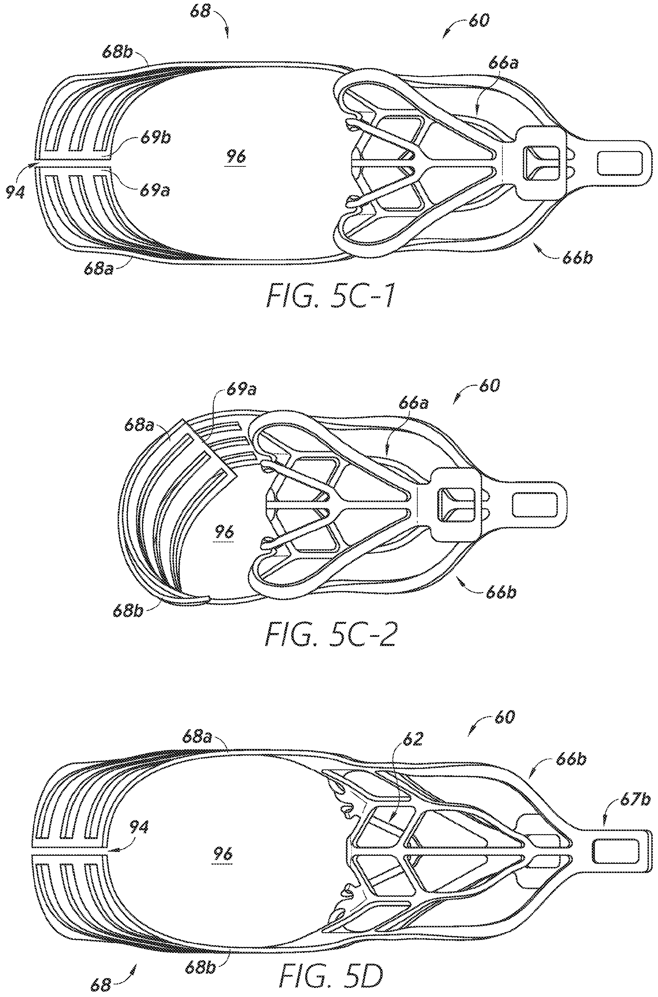

Figure 5D shows an axial view of the shunt structure of Figures 5A and 5B in accordance with one or more examples.

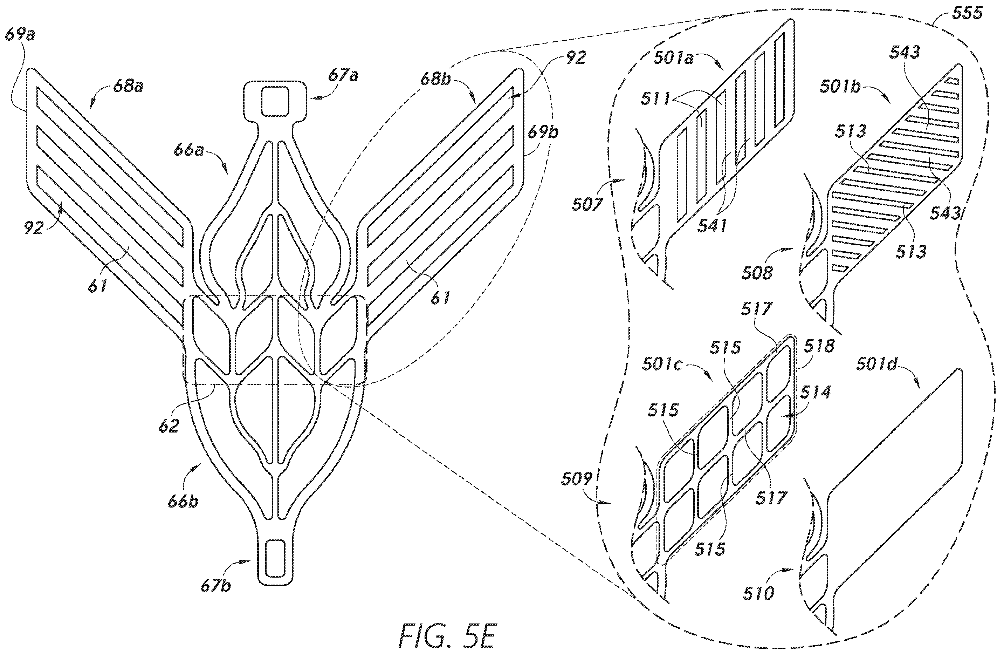

Figure 5E shows the shunt structure of Figure 5A in a flattened configuration, as well as various alternative barrel designs, in accordance with one or more examples.

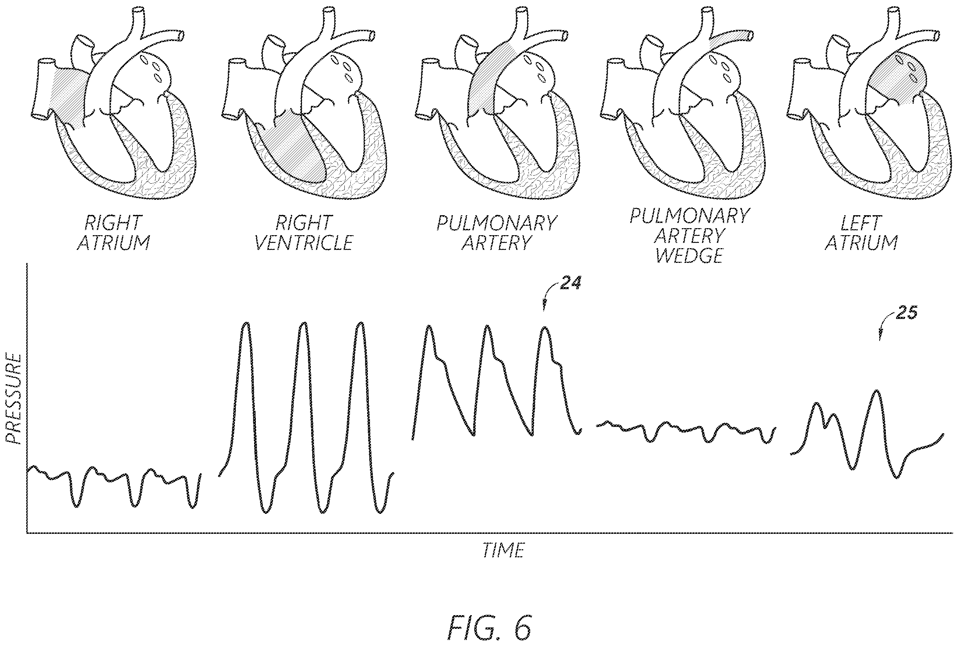

Figure 6 illustrates pressure waveforms associated with various chambers and vessels of the heart according to one or more examples.

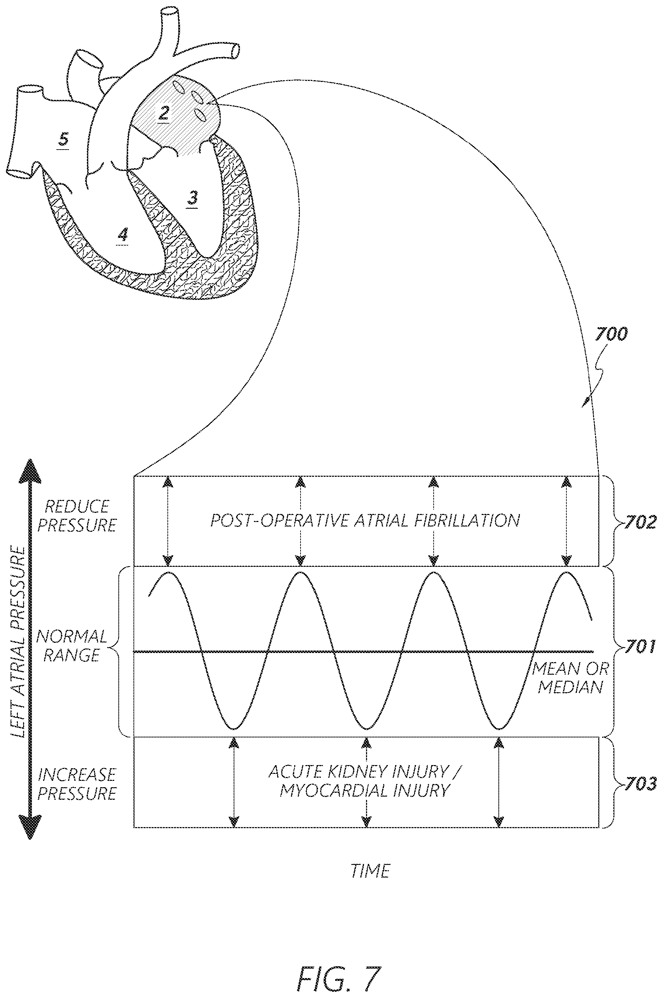

Figure 7 illustrates a graph showing left atrial pressure ranges.

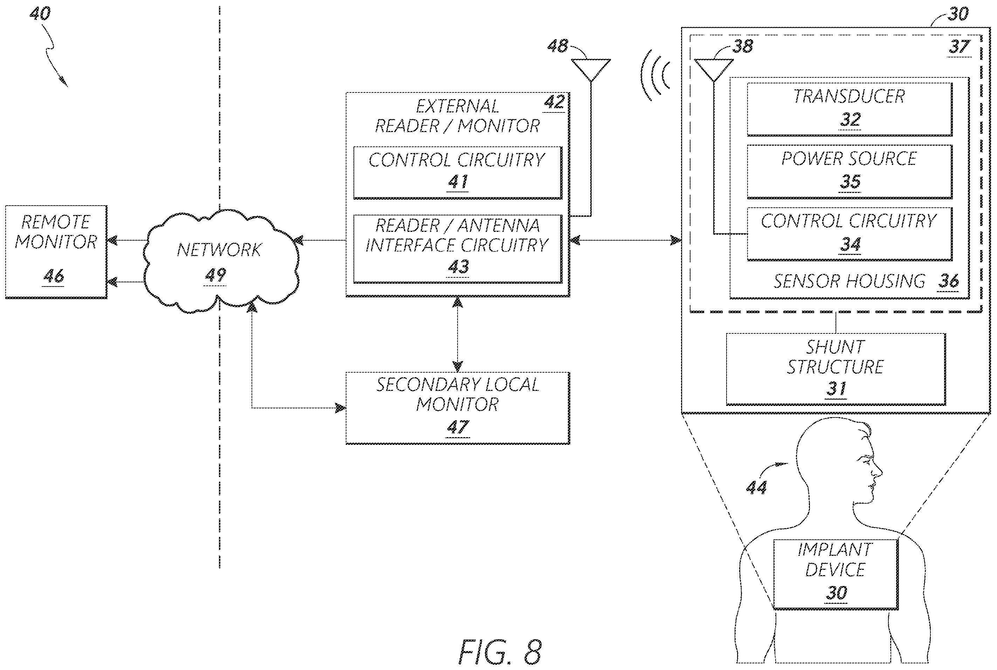

Figure 8 is a block diagram representing a system for monitoring one or more physiological parameters associated with a patient according to one or more examples.

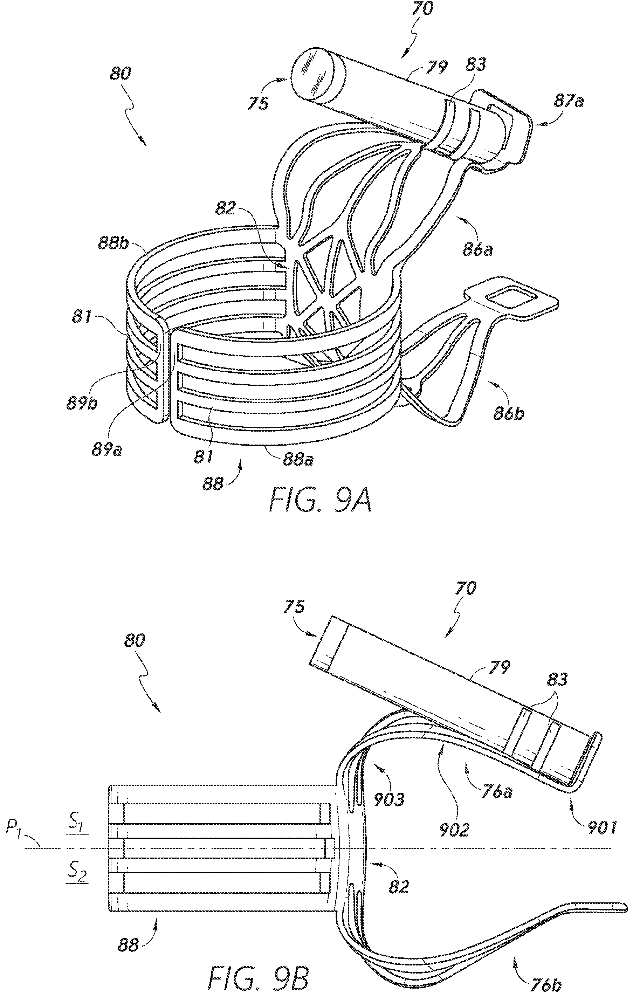

Figures 9A-9D illustrate perspective, side, and axial views, respectively, of a shunt sensor implant device in accordance with one or more examples.

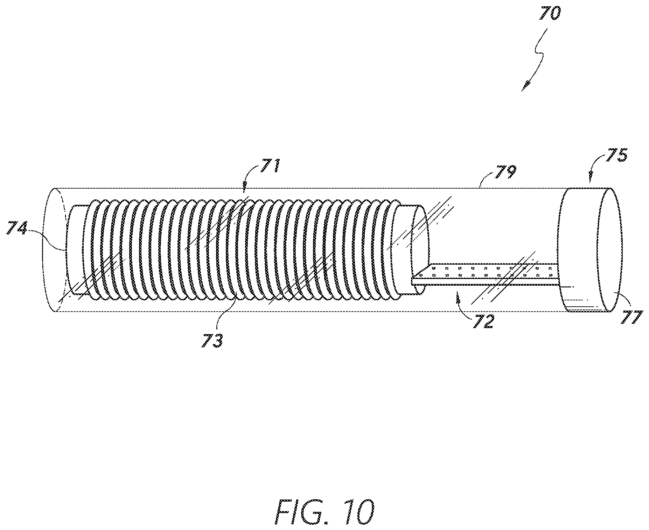

Figure 10 illustrates a sensor assembly/device in accordance with one or more examples.

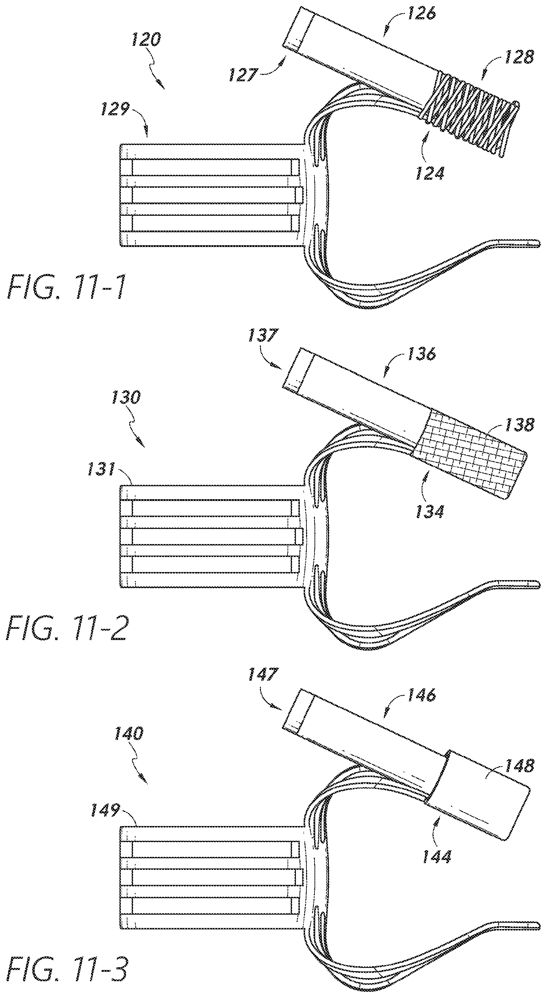

Figures 11-1, 11-2, and 11-3 shows sensor implant devices having various sensor-retention means associated therewith in accordance with one or more examples.

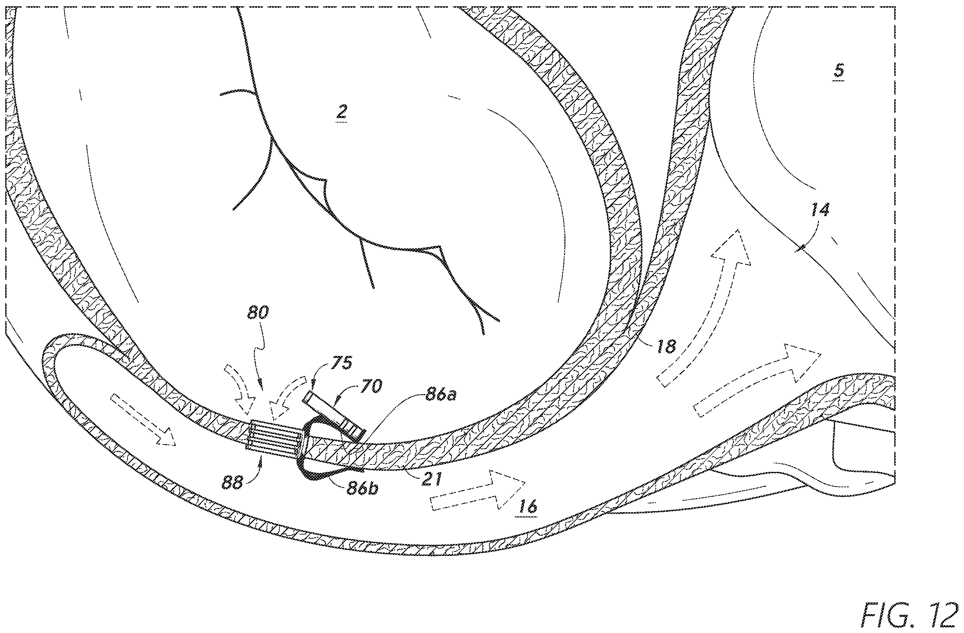

Figure 12 shows a shunt implant device implanted in a coronary sinus tissue wall in accordance with one or more examples.

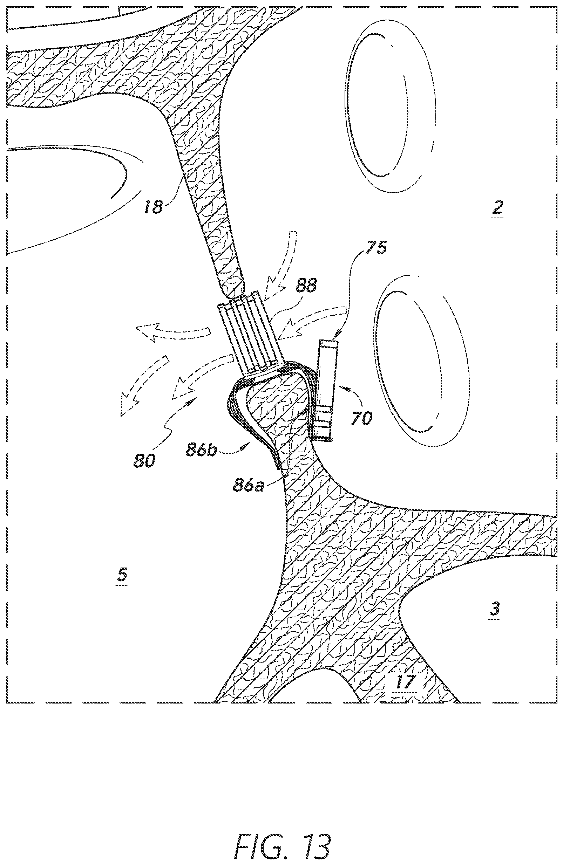

Figure 13 shows a sensor implant device implanted in an atrial septum with a sensor of the device exposed in a left atrium in accordance with one or more examples.

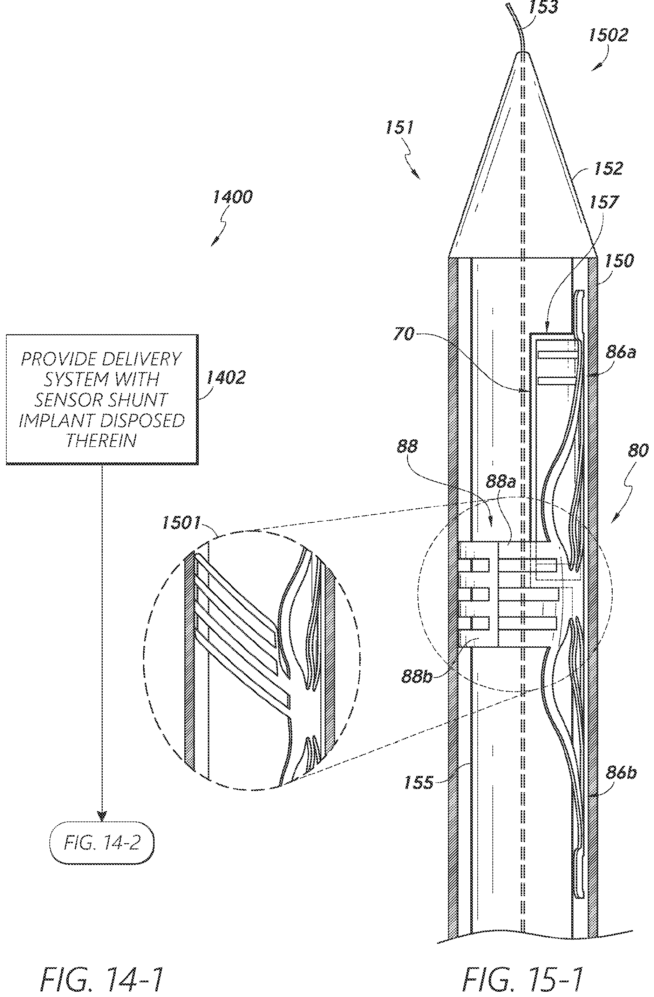

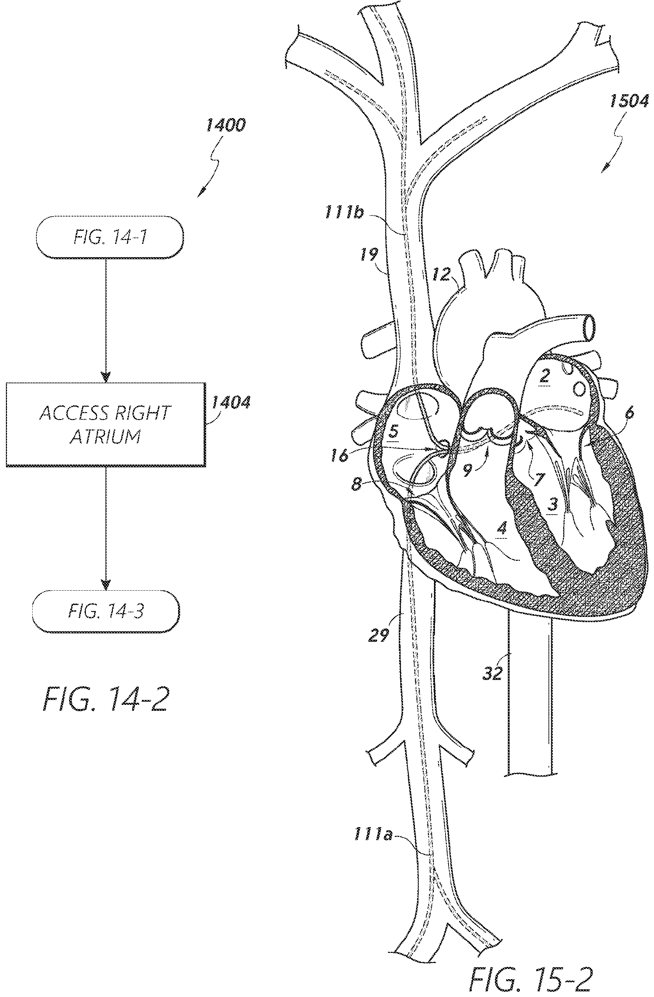

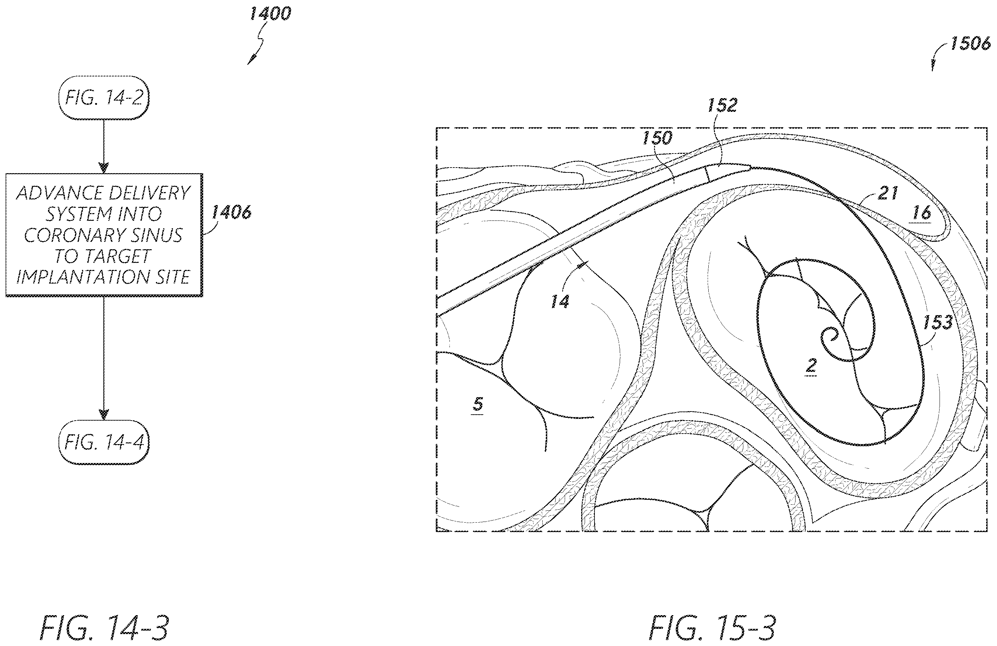

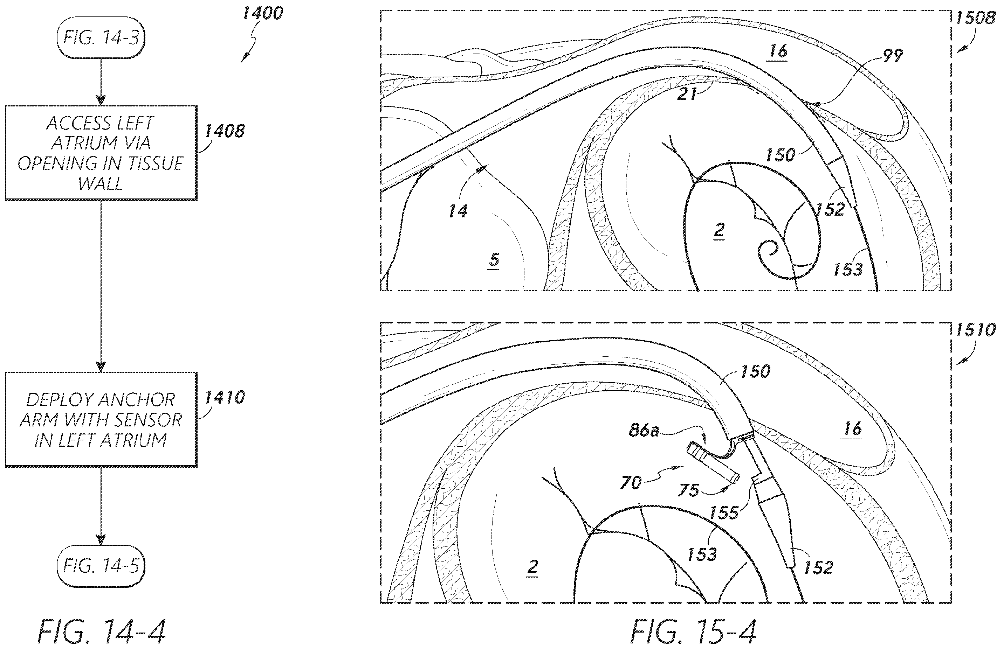

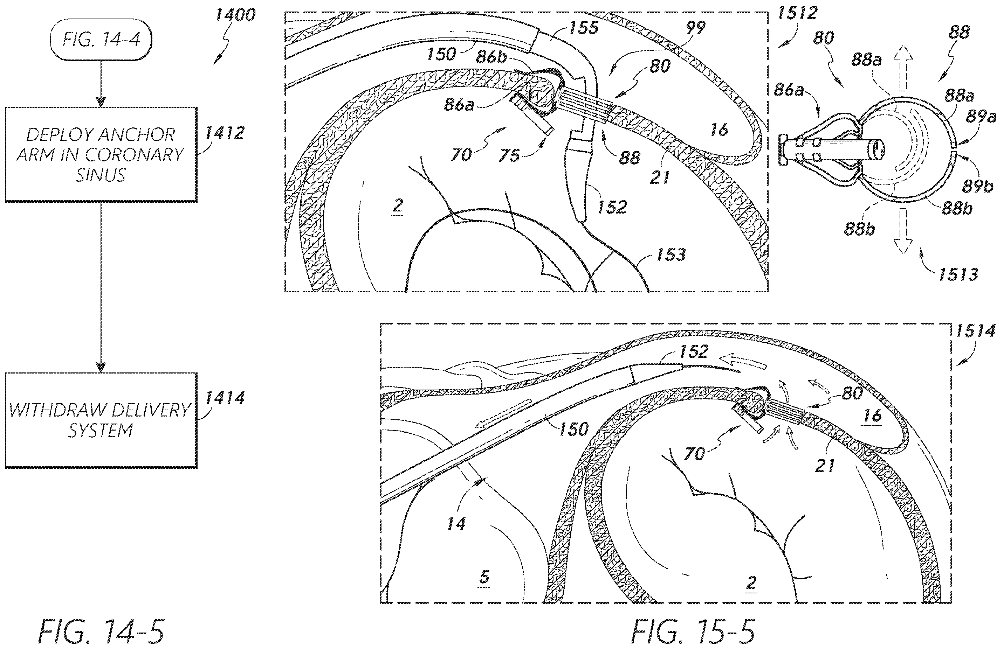

Figures 14-1, 14-2, 14-3, 14-4, and 14-5 provide a flow diagram illustrating a process for implanting a shunt implant device in accordance with one or more examples.
Figures 15-1, 15-2, 15-3, 15-4, and 15-5 provide images of cardiac anatomy and certain devices/systems corresponding to operations of the process of Figures 14-1, 14-2, 14-3, 14-4, and 14-5 in accordance with one or more examples.

### DETAILED DESCRIPTION

The headings provided herein are for convenience only and do not necessarily affect the scope or meaning of the claimed invention.

Although certain preferred examples and examples are disclosed below, inventive subject matter extends beyond the specifically disclosed examples to other alternative examples and/or uses and to modifications and equivalents thereof. Thus, the scope of the claims that may arise herefrom is not limited by any of the particular examples described below. For example, in any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain examples; however, the order of description should not be construed to imply that these operations are order dependent. Additionally, the structures, systems, and/or devices described herein may be embodied as integrated components or as separate components. For purposes of comparing various examples, certain aspects and advantages of these examples are described. Not necessarily all such aspects or advantages are achieved by any particular example. Thus, for example, various examples may be carried out in a manner that achieves or optimizes one advantage or group of advantages as taught herein without necessarily achieving other aspects or advantages as may also be taught or suggested herein.

Methods and structures disclosed herein for treating a patient also encompass analogous methods and structures performed on or placed on a simulated patient, which is useful, for example, for training; for demonstration; for procedure and/or device development; and the like. The simulated patient can be physical, virtual, or a combination of physical and virtual. A simulation can include a simulation of all or a portion of a patient, for example, an entire body, a portion of a body (e.g., thorax), a system (e.g., cardiovascular system), an organ (e.g., heart), or any combination thereof. Physical elements can be natural, including human or animal cadavers, or portions thereof; synthetic; or any combination of natural and synthetic. Virtual elements can be entirely in silica, or overlaid on one or more of the physical components. Virtual elements can be presented on any combination of screens, headsets, holographically, projected, loud speakers, headphones, pressure transducers, temperature transducers, or using any combination of suitable technologies.

Any of the various systems, devices, apparatuses, etc. in this disclosure can be sterilized (e.g., with heat, radiation, ethylene oxide, hydrogen peroxide, etc.) to ensure they are safe for use with patients, and the methods herein can comprise sterilization of the associated system, device, apparatus, etc. (e.g., with heat, radiation, ethylene oxide, hydrogen peroxide, etc.).

Certain reference numbers are re-used across different figures of the figure set of the present disclosure as a matter of convenience for devices, components, systems, features, and/or modules having features that may be similar in one or more respects. However, with respect to any of the examples disclosed herein, re-use of common reference numbers in the drawings does not necessarily indicate that such features, devices, components, or modules are identical or similar. Rather, one having ordinary skill in the art may be informed by context with respect to the degree to which usage of common reference numbers can imply similarity between referenced subject matter. Use of a particular reference number in the context of the description of a particular figure can be understood to relate to the identified device, component, aspect, feature, module, or system in that particular figure, and not necessarily to any devices, components, aspects, features, modules, or systems identified by the same reference number in another figure. Furthermore, aspects of separate figures identified with common reference numbers can be interpreted to share characteristics or to be entirely independent of one another.

Certain standard anatomical terms of location are used herein to refer to the anatomy of animals, and namely humans, with respect to the preferred examples. Although certain spatially relative terms, such as "outer," "inner," "upper," "lower," "below," "above," "vertical," "horizontal," "top," "bottom," and similar terms, are used herein to describe a spatial relationship of one device/element or anatomical structure to another device/element or anatomical structure, it is understood that these terms are used herein for ease of description to describe the positional relationship between element(s)/structures(s), as illustrated in the drawings. It should be understood that spatially relative terms are intended to encompass different orientations of the element(s)/structures(s), in use or operation, in addition to the orientations depicted in the drawings. For example, an element/structure described as "above" another element/structure may represent a position that is below or beside such other element/structure with respect to alternate orientations of the subject patient or element/structure, and vice-versa.

The present disclosure relates to shunt implant devices comprising fluid-passage-forming barrel components configured to be collapsed and/or overlapped over one another in a manner as to allow for a reduced radial profile of the of the barrel the shunt device/structure, which may advantageously facilitate transport of the shunt implant within a delivery system, such as one or more delivery catheters/sheaths. Collapsible-/adjustable-barrel shunt implant devices in accordance with aspects of the present disclosure may include two barrel segments, which are referred to as "wings," "panels," "walls," and/or the like herein, configured to form respective semicircular arcs of a circular or oval flow channel/tube when the shunt implant device is in an expanded state.

In some aspects, the present disclosure relates to systems, devices, and methods for monitoring of one or more physiological parameters of a patient (e.g., blood pressure) using sensor-integrated shunt implant devices. For example, the present disclosure relates to cardiac shunt implant devices that incorporate or are associated with pressure sensors or other sensor devices. The term "associated with" is used herein according to its broad and ordinary meaning. For example, where a first feature, element, component, device, or member is described as being "associated with" a second feature, element, component, device, or member, such description should be understood as indicating that the first feature, element, component, device, or member is physically coupled, attached, or connected to, integrated with, embedded at least partially within, or otherwise physically related to the second feature, element, component, device, or member, whether directly or indirectly.

Certain examples are disclosed herein in the context of cardiac implant devices. However, although certain principles disclosed herein are particularly applicable to the anatomy of the heart, it should be understood that shunt implant devices in accordance with the present disclosure may be implanted in, or configured for implantation in, any suitable or desirable anatomy.

### Cardiac Physiology

The anatomy of the heart is described below to assist in the understanding of certain inventive concepts disclosed herein. In humans and other vertebrate animals, the heart generally comprises a muscular organ having four pumping chambers, wherein the flow thereof is at least partially controlled by various heart valves, namely, the aortic, mitral (or bicuspid), tricuspid, and pulmonary valves. The valves may be configured to open and close in response to a pressure gradient present during various stages of the cardiac cycle (e.g., relaxation and contraction) to at least partially control the flow of blood to a respective region of the heart and/or to blood vessels (e.g., pulmonary, aorta, etc.).

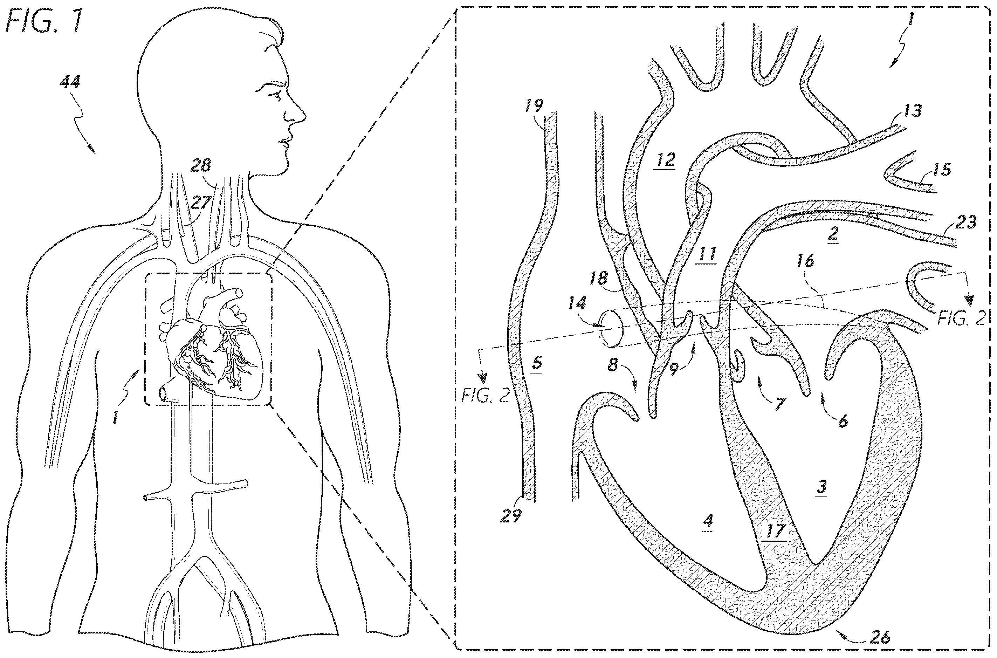

Figure 1 illustrates an example representation of a heart 1 having various features relevant to certain examples of the present inventive disclosure. The heart 1 includes four chambers, namely the left atrium 2, the left ventricle 3, the right ventricle 4, and the right atrium 5. In terms of blood flow, blood generally flows from the right ventricle 4 into the pulmonary artery 11 via the pulmonary valve 9, which separates the right ventricle 4 from the pulmonary artery 11 and is configured to open during systole so that blood may be pumped toward the lungs and close during diastole to prevent blood from leaking back into the heart from the pulmonary artery 11. The pulmonary artery 11 carries deoxygenated blood from the right side of the heart to the lungs.

In addition to the pulmonary valve 9, the heart 1 includes three additional valves for aiding the circulation of blood therein, including the tricuspid valve 8, the aortic valve 7, and the mitral valve 6. The tricuspid valve 8 separates the right atrium 5 from the right ventricle 4. The tricuspid valve 8 generally has three cusps or leaflets and may generally close during ventricular contraction (i.e., systole) and open during ventricular expansion (i.e., diastole). The mitral valve 6 generally has two cusps/leaflets and separates the left atrium 2 from the left ventricle 3. The mitral valve 6 is configured to open during diastole so that blood in the left atrium 2 can flow into the left ventricle 3, and, when functioning properly, closes during systole to prevent blood from leaking back into the left atrium 2. The aortic valve 7 separates the left ventricle 3 from the aorta 12. The aortic valve 7 is configured to open during systole to allow blood leaving the left ventricle 3 to enter the aorta 12, and close during diastole to prevent blood from leaking back into the left ventricle 3.

A wall of muscle, referred to as the septum, separates the left-side chambers of the hear from the right-side chambers. In particular, an atrial septum wall portion 18 (referred to herein as the "atrial septum," "atrial septum," or "septum") separates the left atrium 2 from the right atrium 5, whereas a ventricular septum wall portion 17 (referred to herein as the "ventricular septum," "interventricular septum," or "septum") separates the left ventricle 3 from the right ventricle 4. The inferior tip of the heart 1 is referred to as the apex and is generally located on or near the midclavicular line, in the fifth intercostal space.

The coronary sinus 16 comprises a collection of veins joined together to form a large vessel that collects blood from the heart muscle (myocardium). The ostium of the coronary sinus, which can be guarded at least in part by a Thebesian valve in some patients, is open to the right atrium 5, as shown. The coronary sinus runs along a posterior aspect of the left atrium 2 and delivers less-oxygenated blood to the right atrium 5. The coronary sinus generally runs transversely in the left atrioventricular groove on the posterior side of the heart.

### Health Conditions Associated with Cardiac Pressure and Other Parameters

As referenced above, certain physiological conditions or parameters associated with the cardiac anatomy can impact the health of a patient. For example, congestive heart failure is a condition associated with the relatively slow movement of blood through the heart and/or body, which causes the fluid pressure in one or more chambers of the heart to increase. As a result, the heart does not pump sufficient oxygen to meet the body's needs. The various chambers of the heart may respond to pressure increases by stretching to hold more blood to pump through the body or by becoming relatively stiff and/or thickened. The walls of the heart can eventually weaken and become unable to pump as efficiently. In some cases, the kidneys may respond to cardiac inefficiency by causing the body to retain fluid. Fluid build-up in arms, legs, ankles, feet, lungs, and/or other organs can cause the body to become congested, which is referred to as congestive heart failure. Acute decompensated congestive heart failure is a leading cause of morbidity and mortality, and therefore treatment and/or prevention of congestive heart failure is a significant concern in medical care.

As described above, pressure buildup in one or more chambers or areas of the heart can be associated with congestive heart failure. Therefore, the treatment and/or prevention of heart failure can advantageously involve the alleviation of undesirably high cardiac pressures through the use of shunting or other means. Furthermore, the monitoring of cardiac pressures can be implemented to guide treatment and/or apprise present health conditions. Without direct or indirect monitoring of cardiac pressure, it can be difficult to infer, determine, or predict the presence or occurrence of congestive heart failure. For example, treatments or approaches not involving direct or indirect pressure monitoring may involve measuring or observing other present physiological conditions of the patient, such as measuring body weight, thoracic impedance, right heart catheterization, or the like.

The present disclosure provides systems, devices, and methods for shunting blood from higher (e.g., left-side) chamber(s)/vessel(s) of the heart to relatively lower-pressure chamber(s)/vessels(s) to thereby alleviate high pressure conditions. Furthermore, examples of the present disclosure can provide guidance for the administration of medication relating to the treatment of congestive heart failure at least in part by directly monitoring pressure in the left atrium, or other chamber or vessel for which pressure measurements are indicative of left atrial pressure and/or pressure levels in one or more other vessels/chambers, which can reduce hospital readmissions, morbidity, and/or otherwise improve the health prospects of the patient.

### Cardiac Shunt Implants

Figure 3A shows a perspective view of an example shunt implant device 50 in accordance with one or more examples. Figure 3B shows a side view of the example shunt device/structure 50 of Figure 3A, including a detailed view 342 of a barrel portion 58 of the shunt structure 50. Figures 3C-1 and 3C-2 show axial views of the example shunt device/structure 50 of Figures 3A and 3B with the barrel 58 thereof in expanded and compressed configurations, respectively. Figure 3D shows the example shunt device/structure 50 of Figure 3A in a flattened configuration in accordance with one or more examples.

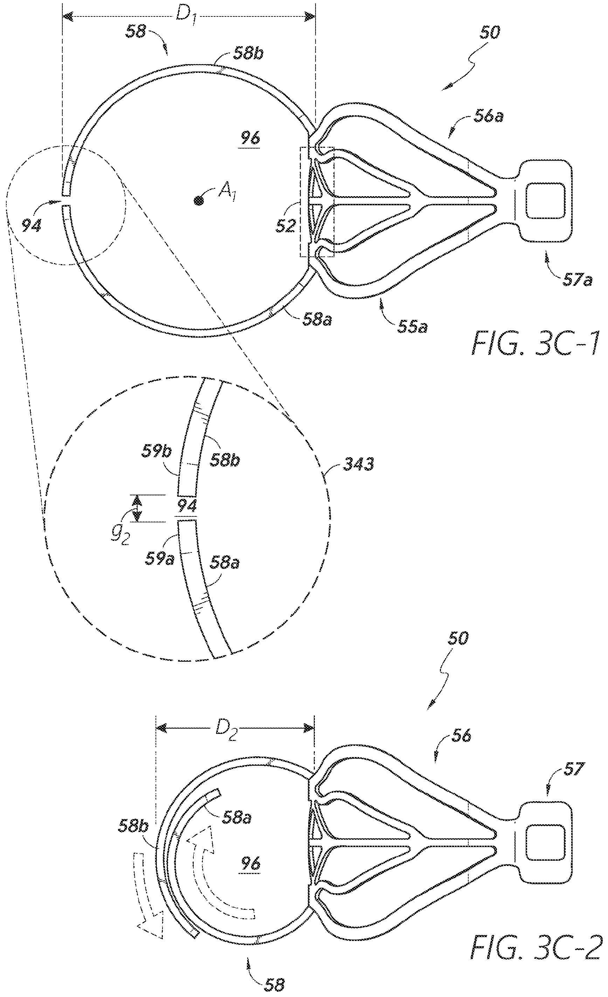

The shunt structure/device 50 represented in Figures 3A-3D may represent an example of a cardiac implant device, which may or may not be integrated with pressure sensor functionality in accordance with certain examples disclosed herein. The shunt device/structure 50 has an expandable and compressible barrel 58, wherein compression of the barrel 58 can facilitate placement within a transport catheter/sheath for delivery to the target implantation site. When expanded, as shown in Figures 3A, 3B, and 3C-1, a central flow channel 96 of the shunt 50 may define a generally circular or oval opening/channel. The channel 96 may be configured to hold the sides of a puncture opening in a tissue wall to form a blood flow path between chamber(s) and/or vessel(s) of the heart that are separated by the tissue wall. For example, the shunt 50 may be configured to be implanted in the wall separating the coronary sinus and the left atrium, and/or in the interatrial septum. Although described in some contexts as being placed in a single tissue wall, it should be understood that shunt devices disclosed herein can be implanted in multiple (e.g., parallel) tissue walls, thereby providing a flow path through such multiple walls. For example, the anchor arms 56 of the shunt 50 (or other shunt devices disclosed herein) can pinch/hold tissue walls together to allow for shunting through multiple walls.

The central flow channel 96 may be partly formed by a pair of arcuate panels/wings 58a, 58b that emanate from a backbone/support portion 52 of the shunt structure that forms a portion of the flow channel/barrel 58, wherein such portion 52 may generally be positioned/disposed circumferentially opposite of a gap 94 and/or contact interface between the distal struts/edges 59a, 59b of the barrel panels/wings 58a, 58b when the barrel is formed/expanded as shown in Figure 3A. The term "wing" is used herein according to its broad and ordinary meaning, and may refer to any type of structure or form that projects or extends from a support or other structure of a shunt structure/device, including any type of panel, wall, tab, strap, band finger, arm, flap, or the like. For example, a "wing" or "panel" of a shunt structure as described herein may extend/project generally perpendicularly from a primary dimension *d₁* of one or more anchor arms of the shunt structure with respect to a flattened-out view/configuration of the shunt as shown in Figure 3D. Furthermore, a "wing" or "panel" of a shunt structure may extend/project generally perpendicularly from an axial dimension *d₁* of a backbone/barrel-support portion of the shunt structure from which one or more anchor arms and/or the wing(s)/panel(s) emanate/project.

The barrel wings 58a, 58b can have any suitable or desirable structural form, as described in detail below. For example, the wings 58a, 58b can be formed of a generally parallel arrangement of elongate thin struts 51 separated by gap distances *g₁* that form an array of parallel slits, cells, or openings 92. In some examples, the barrel wings/panels 58a, 58b (and/or the entire shunt structure 50) is/are formed by super-elastic struts that are configured to be compressed, curved, expanded, manipulated, or the like, such as for fit/placement in a delivery catheter. In some examples, the shunt structure 50 comprises shape memory metal (e.g., nitinol) configured to be compressed for delivery and automatically expand to the expanded configuration shown in Figure 3A when released from delivery system constraint(s).

Formation of the shunt 50 using a plurality of interconnected struts forming cells therebetween may serve to at least partially increase the flexibility of the shunt, thereby enabling compression thereof and expansion at the implant site. The interconnected struts 51 of the barrel 58 advantageously provide a cage structure having sufficient rigidity and structure to hold the tissue at the puncture in an open position. The backbone/support portion 52 of the shunt structure 50 / barrel 58 serves to connect the wings/panels 58a, 58b and extend axially between distal and proximal anchor arms 56a, 56b on each axial side of the barrel 58. The barrel wings/panels 58a, 58b and backbone portion/support 52 together may define a tubular lattice, as shown, forming a cylindrical tubular form/barrel, which may include an axial gap 94 between distal/end edges/struts 59 of the respective wings 58a, 58b. The backbone support portion 52 includes one or more axial struts 158, 159 and/or one or more angled/diagonal (relative to the flow axis/dimension *A₁*/*d₁* of the barrel 58) struts 157, as shown. Figure 3C-1 shows the backbone support portion 52 of the shunt 50 as diametrically opposite the gap/interface 94 between the ends/edges of the barrel wings 58a, 58b.

Although certain examples of shunts disclosed herein comprise flow channels having substantially circular cross-sections as shown in Figure 3C-1, in some examples, shunt structures in accordance with the present disclosure have oval-shaped, rectangular, diamond-shaped, or elliptical flow channel configuration. For example, relatively elongated barrel wings compared to the illustrated configuration of Figures 3A-3D may produce a rectangular or oval-shaped flow channel when the edges thereof are brought together. Such shapes of shunt flow channels may be desirable for larger punctures, while still being configured to collapse down to a relatively small delivery profile.

Each of the distal and proximal anchor arms 56a, 56b is configured to curl outward from the backbone support portion 52 and be set to point approximately radially away from the axis *A₁* of the central flow channel 96 in the expanded configuration. The expanded flanges/arms 56a, 56b may serve to secure the shunt 50 to a target tissue wall. The anchor arms 56a, 56b, as shown may not be annular/circular around the circumference/perimeter of the barrel 58, but instead may extend outward generally in only one plane, as shown (e.g., parallel to the horizontal/lateral tissue plane *p₁*)*.* Additional aspects and features of shunt, implant, and/or anchor structures that may be embodied in examples of the present disclosure are disclosed in U.S. Pat. No. 9,789,294, entitled "Expandable Cardiac Shunt," issued on October 17, 2017.

As shown in Figure 3B, the expanded shunt 50 can have an overall height *h₁* of between about 5-10 mm, such as about 6.7±1.0 mm. In some examples, the barrel 58 has a height *h₂* of between 3-5 mm, such as about 3.9±0.2 mm. As shown in the detailed image 342 of Figure 3B, the spacing *g₁* between the struts 51 of the barrel 58 that define the slits/cells 92 can be between 0.5-2 mm, such as about 1 mm. The thickness *t₁* of the struts 51 that define the slits/cells 92 can be at least 0.2 mm, such as between 0.2-0.5 mm.

With reference to Figures 3B and 3D, the barrel wings 58a, 58b may have terminal edges/struts 59a, 59b that are parallel to the axis *A₁* of the barrel 58, or may alternatively be angled (as shown in dashed line in Figures 3B and 3D), such that a distal portion of the barrel 58 has a greater diameter than a proximal portion thereof, which may provide a funneling effect/structure for funneling shunted blood through the barrel 58.

As shown in the views of Figure 3C-1 and 3D, the anchor arms 56 can have a somewhat triangular plan view shape with a wide base at the central flow tube 96 narrowing to an apex at the terminal ends 57. The struts that form the anchor arms 56 can be designed so that they easily collapse into a compact size that fits into a delivery catheter/sheath. The shunt 50 can be symmetrical across the horizontal plane (e.g., tissue plane) *p₁,* which can represent an axial midplane of the shunt 50. For example, the distal anchor arm 56a can be generally the same size and shape as the proximal anchor arm 56b. In some examples, the length of the anchor arms 56 is about 7.0 mm.

The anchor arms 56 on each side of the barrel/tube 58 can converges toward each other so that their terminal ends 57 are closer together than the medial portions 55 of the arms when the arms 56 are expanded/extended as shown in Figures 3A and 3B. For example, the terminal ends 57 of the arms 56 can be spaced relatively close in the expanded configuration so that they flex toward the axial center *p₁* of the shunt 50 to grip the tissue wall in which the shunt 50 is implanted (e.g., generally in-line with the plane *p₁*), thus helping to maintain the shunt 50 in place. In some examples, super-elastic characteristics of the arms 56 can prevent the arms 56 from applying excessive clamping forces to the tissue wall, which could potentially cause necrosis or other damage. The terminal ends 157 of the anchor arms 56 can define certain aperture features or other closed shapes so as to be configured to be engaged by actuating rods or other engagement/manipulation means.

Slits 92 formed between adjacent parallel struts 51 of the respective barrel wings 58a, 58b can extend a majority of the length *l₁* of the wings/panels 58, as shown. Although three parallel slits 92 are shown, separated/defined by four parallel struts 51, it should be understood that barrel wings/panels in accordance with aspects of the present disclosure may include any number or arrangement of slits/struts (e.g., parallel slits/struts), such as four slits defined by five struts, or other numbers.

The cutouts/slits 92 between the lateral/circumferential struts 51 can provide increased flexibility for the barrel wings/panels 58a, 58b to provide the desired curvature of the barrel 58. The slits 92 can be formed by cutting out strips of material of the barrel walls/panels 58a, 58b such as through laser cutting or the like. When the barrel 58 expands within an opening in a target tissue wall, to thereby form a shunt channel through the tissue wall, the slits 92 may allow for tissue of the tissue wall to protrude into the diameter/space of the barrel 58 through the slits 92, thereby further securing the barrel 58 to the tissue wall. In addition, tissue ingrowth may be facilitated/permitted with respect to the tissue disposed in, and/or protruding into, the slits 92, wherein such tissue ingrowth may cross over one or more of the struts 51 on an inner diameter of barrel 58, thereby further securing the shunt 50 to the tissue wall. Such tissue ingrowth may advantageously be sufficient to secure the barrel 58 to the tissue wall, while not overly encroaching into the flow channel 96 or occluding the channel 96 or otherwise reducing the orifice of the shunt barrel 58 to a detrimental degree.

The barrel wings/panels 58a, 58b are configured to form the arcuate walls of the barrel 58, wherein terminal/distal ends/edges 59a, 59b of the respective barrel wings/panels are brought into proximity with one another to close the tubular form of the barrel 58 as shown in Figure 3A. The terminal/distal edges/struts 59a, 59b of the respective barrel wings/panels 58a, 58b, in the expanded configuration, can contact/press against one another, and/or can be configured/positioned such that a gap 94 having a distance *g₂* (see detail 343 in Figure 3C-1) is present between the wing/panel edges. Where present, the gap distance *g₂* of the gap 94 between the distal/terminal portions/edges of the barrel wings/panels 58a, 58b may advantageously be narrow/small enough as to allow for the barrel wings/panels to maintain the channel 96 and/or prevent substantial encroachment into the inner diameter of the barrel 58 by biological tissue through the gap 94.

Figures 3C-1 and 3C-2 show axial views of the shunt 50 in fully expanded and compressed/overlapped configurations, respectively. For example, as referenced above, the image of Figure 3C-1 shows the expanded barrel 58, wherein the barrel wings/panels 58a, 58b form arcuate segments of a common circumference of the barrel 58. When expanded, radial alignment/overlapping of the terminal edges/struts 59a, 59b of the respective barrel wings/panels 58a, 58b can prevent circumferential/inward compression/overlapping of the barrel walls/wings and/or distal/terminal portions thereof. For example, the radial alignment of the terminal ends/edges 59a, 59b and/or associated struts (e.g., axial struts of the barrel 58) can cause an interference lock preventing circumferential overlapping of the edges 59a, 59b of the respective barrel wings/panels 59a, 59b. In the expanded configuration shown in Figure 3C-1 , the edges 59a, 59b of the barrel wings 58a, 58b are radially overlapped, and thus cannot deflect inwardly and/or circumferentially past one another without relative radial deflection of one or both of the wings/panels 58a, 58b.

Figure 3C-2 shows a radially and circumferentially compressed and circumferentially overlapped configuration of the barrel wings/panels 58a, 58b, which may be implemented for the purpose of reducing the diametrical profile *d₂* of the barrel 58 for the purpose of transporting the shunt 50 within a particular delivery system (e.g., catheter/sheath). In the configuration shown inFigure 3C-2 , distal portions of the barrel wings/panels 58a, 58b are circumferentially overlapped. The compression shown in Figure 3C-2 may be achieved by radially displacing one or more of the barrel wings 58a, 58b relative to the other and circumferentially/inwardly (e.g., towards the axis *A₁* of the barrel 58) deflecting the wing(s) to cause distal/terminal portions thereof to overlap as shown in Figure 3C-2 . Such overlapping may generally reduce the cross-sectional area of the conduit/channel 96 formed by the barrel 58, and likewise reduce the diameter/profile of the barrel 58. For example, the compressed diameter *d₂* may be generally less than the expanded diameter *d₁* shown with respect to the configuration of Figure 3C-1 . Therefore, by inwardly curling/deflecting the barrel wings/panels in a circumferentially overlapping fashion as shown in Figure 3C-2 , the shunt 50 can be configured to fit in a relatively smaller profile, advantageously allowing for transport of the shunt 50 to target anatomy through relatively narrow and/or tortuous access paths, such as within portions of the patient's vasculature. The curling/compression of the barrel 58 may further be implemented to accommodate a shunt opening in a tissue wall that is of a relatively small size. That is, the ability to circumferentially overlap the barrel wings/panels 58a, 58b can allow for customization of the barrel size to a desired diameter and/or area to fit a target opening. In such implementations, it may be desirable to implement a mechanism for locking relative positions of the barrel wings/panels at the desired compressed/overlapped configurations to prevent further compression or expansion of the barrel 58 after implantation. For example, the wings/panels may be locked to one another in some manner (e.g., pin, ratchet mechanism, clamp, hook, or other locking means) when the desired compression state is achieved.

Figure 3D shows a flattened view of the expandable shunt 50, with the barrel wings/panels 58a, 58b and the anchor arms 56a, 56b extended straight outward/away from the backbone portion 52 of the shunt structure. The various struts that form the shunt 50 can be fabricated by laser-cutting a memory metal (e.g., nitinol) tube. For example, the tube can have a wall thickness of between about 0.1-0.3 mm, such as about 0.2 mm.

Although examples are disclosed herein of shunt devices that comprise a single pair of axially-opposite anchor arms, as shown in Figure 3A, it should be understood that examples of the present disclosure may comprise one or more additional anchor arms. For example, with respect to shunt devices having a pair of barrel wings/panels configured to circumscribe a flow channel/barrel, wherein terminal/distal edges of such wings/panels can be brought into proximity with one another in order to provide a cylindrical/tubular form of the barrel/channel, one or more additional anchor arms 356 may be associated with and/or emanate from a distal 357 or medial 358 lengthwise portion of a respective barrel wing/panel. For example, Figure 3D shows example positions of additional anchor arms 356, shown in dashed form, wherein such arm(s) may be disposed on either or both axial side(s) of the barrel 58. Furthermore, such arm(s) may be circumferentially aligned with one another and emanate from a common barrel wings/panel, or may be circumferentially offset and/or emanate from separate barrel wings/panels, as shown in dashed in Figure 3D.

Figures 4A-4D show side views of example shunt structures having various barrel designs in accordance with a plurality of examples. For example, although Figures 3A-3D illustrate a shunt device 50 having a barrel 58 formed of barrel wings/panels 58a, 58b that have circumferential/lateral struts forming elongated circumferential/ lateral slits therein, it should be understood that barrel wings/panels in accordance with aspects of the present disclosure may have any suitable or desirable structural, strut, and/or cell design. For example, Figure 4A shows a shunt device 50a having barrel wings/panels 401a that comprise vertical/axial struts 441 and/or slits/cells 411. Such configuration may provide advantageous curving flexibility for the wings/panels 401a, while also providing desirable axial rigidity.

Figure 4B shows a shunt device 50b having barrel wings/panels 401b that comprise diagonal struts 443 and/or slits/cells 413. Figure 4C shows a shunt device 50c having barrel wings/panels 401c that comprise vertical 415 and lateral/horizontal 417 struts, which form rectangular (e.g., square) cells 414. In some examples, the structure of the barrel wings/panels forms a substantially contiguous wall surface through at least a portion of the barrel, as shown in Figure 4D, wherein the illustrated shunt 50d includes barrel wings/panels without slits or cells cut therein over at least a circumferential portion thereof.

Figures 3A-3D and 4A-4D (as well as other examples disclosed herein) show shunt devices having barrel wings/panels that, when distal struts/edges thereof are brought together, form a cylindrical form having an axis *A₁* that is generally perpendicular/orthogonal to the horizontal/lateral tissue plane *p₁* associated with the shunt device and/or tissue wall in which the shunt device is implanted. However, it should be understood that collapsible-barrel shunt devices as disclosed herein can have barrel wings/panels that come together to form shunt conduits/channels that have axes oriented at any suitable or desirable angle relative to the horizontal/tissue plane of the respective shunt device. That is, examples of the present disclosure can comprise barrel wings/panels that form angled/canted barrels/conduits relative to the tissue walls in which they are configured to be implanted (e.g., relative to tissue-holding plane of the anchor arm(s) of the shunt device).

Figure 5A shows a perspective view of an example shunt device/structure 60 having a canted/angled barrel 68 in accordance with one or more examples. Figure 5B shows a side view of the shunt device/structure 60 of Figure 5A, as well as various alternative canted barrel designs. Figures 5C-1 and 5C-2 show axial views of the example shunt structure 60 of Figures 5A and 5B with the barrel thereof in expanded and compressed configurations, respectively. Figure 5D shows an axial view of the example shunt structure of Figures 5A and 5B. Figure 5E shows the example shunt structure of Figure 5A in a flattened configuration, as well as various alternative barrel designs, in accordance with one or more examples. The following disclosure relates to Figures 5A-5E.

The shunt device 60 includes barrel wings/panels 68a, 68b and a backbone/support portion/structure 62, which together, when the distal edges/struts 69a, 69b are brought together or in proximity to one another (e.g., within 2 mm) such that the wings/panels 68a, 69a have arcuate forms as shown, define a tubular lattice forming a barrel/channel that is angled (e.g., "canted," or "tilted") relative to the central/tissue plane *p₁* associated with the shunt device 60 and shown in Figure 5B.

The barrel backbone/support portion 62 can comprise one or more struts, which may be angled in a manner similar to the axis *A₂* of the barrel 68. For example, the barrel axis *A₂* and/or the strut(s) of the backbone/support portion 62 of the barrel 68 may extend at acute angles *θ₁*, *θ₂* relative to the tissue plane *p₁* and the perpendicular axis *A₃* through the flow channel 96 of the barrel 68. That is, as seen in Figure 5B, an imaginary reference axis *A₃* may be drawn generally perpendicular to the horizontal/tissue reference plane *p₁,* such that the angled axis *A₂* is defined by the angled backbone portion 62 of the barrel 96 and the wings/panels 68a, 68b (e.g., wing edges/struts 69a, 69b). Indeed, the central flow tube/channel 96 can extend at the angle *θ₂* from the perpendicular axis *A₃.* The angle *θ₂* may be between 30-60° in some examples, such as about 45°. The horizontal tissue plane *p₁* is generally defined by an area of the tissue wall (e.g., wall between the coronary sinus and the left atrium) in which the shunt implant device 60 is configured to be implanted in the immediate vicinity of the barrel 68 when the device 60 is implanted (e.g., an imaginary plane in the context of a shunt device that is not implanted).

Although the barrel 68 is oriented at an angle *θ₂*, the opening as seen in Figures 5C-1 and 5D formed by the barrel 68 may be considered generally perpendicular to the plane *p₁* and, when implanted in a target tissue wall, can permit direct blood flow between the chamber(s)/vessel(s) joined by the shunt device 60. For example, the angled barrel 68 can be wide and short enough such that proper shunting occurs as if the barrel 68 were essentially perpendicular to the tissue plane *p₁.* The struts 61 of the barrel wings/panels 68a, 68b can define a tubular or circular lattice. As with other examples presented herein, the struts, as shown, may not form a contiguous wall surface, but rather may form open cells 92 (e.g., elongate circumferential/lateral slits). The tilt *θ₂* of the collapsible shunt 60 can facilitate collapsing of the barrel 68 for placement into a delivery catheter, and further the expansion of the anchor arms 66 on both axial sides of the target tissue wall. The angled configuration of the barrel 68 can result in a horizontal/lateral offset *d₂* of the tissue-contact pads/feet 67 of the anchor arms 66. Such lateral tissue-contact offset can advantageously reduce direct pinching of the tissue wall between the tissue-contact pads/feet 67a, 67b.

In the illustrated example of Figure 5A, the barrel wings/panels 68a, 68b comprise circumferential/lateral struts 61 separated by elongated circumferential/lateral slits/cells 92. However, as with other disclosed examples, variations on barrel wing/panel strut design are possible. For example, Figures 5B and 5D show side views of example shunt structures having various angled/canted barrel wing/panel designs in accordance with a plurality of examples. The images 505, 555 of Figures 5B and 5D, respectively, show a shunt device 507 having barrel wings/panels 501a that comprise angled vertical/axial struts 541 and/or slits/cells 511. Such configuration may provide advantageous curving flexibility for the wings/panels 501a, while also providing desirable axial rigidity.

Images 505, 555 further show a shunt device 508 having barrel wings/panels 501b that comprise diagonal struts 543 and/or slits/cells 513. Images 505, 555 further show a shunt device 509 having barrel wings/panels 501c that comprise vertical 515 and lateral/horizontal 517 struts, which form parallelogram-shaped (e.g., diamond) cells 514. Thus, the barrel wings/panels 501c are defined by a generally parallelogram arrangement of struts that forms an array of parallelogram-shaped cells or openings 514. The side walls 501c are generally circumscribed by a larger parallelogram 518 that is tilted in the same direction as the tilted axis *A₂* through the barrel 68. Indeed, each of the cells 514 is tilted in the same direction. In some examples, as shown, there may be two (or other number) rows of four (or other number) cells 514 stacked along the barrel axis *A₂* that are laterally offset lengthwise from each other.

In some examples, the structure of the barrel wings/panels forms a substantially contiguous wall surface through at least a portion of the barrel, as shown in images 505, 555 of Figures 5B and 5D, wherein the illustrated shunt 510 includes barrel wings/panels 501d without slits or cells cut therein over at least a circumferential portion thereof.

The tilt of the shunt structure 60 may facilitate collapse of the shunt 60 into a delivery catheter, as well as the expansion of the anchor arms 66 on both sides of a target tissue wall. The anchor arms 66 may transition in and out of alignment with the angled barrel axis *A₂* between the collapsed and expanded states of the shunt 60. For angled-/canted-barrel examples, the anchor arms 66 can include a relatively longer anchor arm 66b (e.g., proximal) that extends radially outwardly from the barrel 68 when released from the delivery system, but to a lesser extent than the opposite (e.g., distal) anchor arm 66a, which may be relatively shorter than the anchor arm 66b. For example, the shorter arm 66a may expand by rotating outward more than 90°, while the longer arm 66b may rotate/expand outward less than 90°.

### Cardiac Pressure Monitoring

While examples of shunt devices disclosed herein can be implemented/configured without any pressure (or other-type) sensor functionality, it should be understood that any of the shunt examples disclosed herein can include integrated sensor functionality, which may provide various benefits. For example, cardiac pressure monitoring in accordance with examples of the present disclosure may provide a proactive intervention mechanism for preventing or treating congestive heart failure and/or other physiological conditions. Generally, increases in ventricular filling pressures associated with diastolic and/or systolic heart failure can occur prior to the occurrence of symptoms that lead to hospitalization. For example, cardiac pressure indicators may present weeks prior to hospitalization with respect to some patients. Therefore, pressure monitoring systems in accordance with examples of the present disclosure may advantageously be implemented to reduce instances of hospitalization by guiding the appropriate or desired titration and/or administration of medications before the onset of heart failure.

As referenced above, among cardiac pressures, pressure elevation in the left atrium may be particularly correlated with heart failure. Figure 6 illustrates example pressure waveforms associated with various chambers and vessels of the heart according to one or more examples. The left atrial pressure waveform 25, among the various cardiac pressure waveforms, may be considered to provide the best feedback for early detection of congestive heart failure. Furthermore, there may generally be a relatively strong correlation between increases and left atrial pressure and pulmonary congestion.

Left atrial pressure may generally correlate well with left ventricular end-diastolic pressure. However, although left atrial pressure and end-diastolic pulmonary artery pressure can have a significant correlation, such correlation may be weakened when the pulmonary vascular resistance becomes elevated. That is, pulmonary artery pressure generally fails to correlate adequately with left ventricular end-diastolic pressure in the presence of a variety of acute conditions, which may include certain patients with congestive heart failure. Therefore, pulmonary artery pressure measurement alone, as represented by the waveform 24, may be an insufficient or inaccurate indicator of left ventricular end-diastolic pressure, particularly for patients with co-morbidities, such as lung disease and/or thromboembolism. Left atrial pressure may further be correlated at least partially with the presence and/or degree of mitral regurgitation. Left atrial pressure readings may be relatively less likely to be distorted or affected by other conditions, such as respiratory conditions or the like, compared to the other pressure waveforms shown in Figure 6. Generally, left atrial pressure may be significantly predictive of heart failure, such as up two weeks before manifestation of heart failure.

Cardiac pressure monitoring, such as left atrial pressure monitoring, can provide a mechanism to guide administration of medication to treat and/or prevent congestive heart failure. Such treatments may advantageously reduce hospital readmissions and morbidity, as well as provide other benefits. An implanted pressure-sensor-integrated shunt implant device in accordance with examples of the present disclosure may be used to predict heart failure up two weeks or more before the manifestation of symptoms or markers of heart failure (e.g., dyspnea). When heart failure predictors are recognized using cardiac pressure sensor examples in accordance with the present disclosure, certain prophylactic measures may be implemented, including medication intervention, such as modification to a patient's medication regimen, which may help prevent or reduce the effects of cardiac dysfunction. Direct pressure measurement in the left atrium can advantageously provide an accurate indicator of pressure buildup that may lead to heart failure or other complications. For example, trends of atrial pressure elevation may be analyzed or used to determine or predict the onset of cardiac dysfunction, wherein drug or other therapy may be augmented to cause reduction in pressure and prevent or reduce further complications.

Figure 7 illustrates a graph 700 showing left atrial pressure ranges including a normal range 701 of left atrial pressure that is not generally associated with substantial risk of adverse health conditions. Examples of the present disclosure provide systems, devices, and methods for determining whether a patient's left atrial pressure is within the normal range 701, above the normal range 703, or below the normal range 702 through the use of certain sensor implant devices. For detected left atrial pressure above the normal range, which may be correlated with an increased risk of heart failure, examples of the present disclosure as described in detail below can inform efforts to reduce the left atrial pressure until it is brought within the normal range 701. Furthermore, for detected left atrial pressure that is below the normal range 701, which may be correlated with increased risks of acute kidney injury, myocardial injury, and/or other health complications, examples of the present disclosure as described in detail below can serve to facilitate efforts to increase the left atrial pressure to bring the pressure level within the normal range 701.

### Sensor-Integrated Shunt Implant Devices and Systems

Figure 8 shows a system 40 for monitoring one or more physiological parameters (e.g., left atrial pressure and/or volume) in a patient 44 using a sensor-integrated shunt device 30 according to one or more examples. For example, the shunt implant device 30 can be implanted in the patient's heart, or associated physiology. In some implementations, the shunt implant device 30 can be implanted at least partially within the left atrium and/or coronary sinus of the patient's heart.

In some examples, the implant device comprises certain shunt structure 31, such as including two barrel-forming wings/panels and a plurality of anchor arms, as described herein. The shunt structure 31 can be physically integrated with and/or connected to a sensor device 37. The sensor device 37 may be, for example, a pressure sensor, or other type of sensor. In some examples, the sensor 37 comprises a transducer 32, such as one or more microelectromechanical system (MEMS) devices (e.g., MEMS pressure sensors, or other type of sensor transducer), as well as certain control circuitry 34, which may be embodied in, for example, one or more application-specific integrated circuits (ASIC).

The control circuitry 34 may be configured to process signals received from the transducer 32 and/or communicate signals associated therewith wirelessly through biological tissue using the antenna 38. The term "control circuitry" is used herein according to its broad and ordinary meaning, and may refer to any collection of processors, processing circuitry, processing modules/units, chips, dies (e.g., semiconductor dies including come or more active and/or passive devices and/or connectivity circuitry), microprocessors, microcontrollers, digital signal processors, microcomputers, central processing units, field programmable gate arrays, programmable logic devices, state machines (e.g., hardware state machines), logic circuitry, analog circuitry, digital circuitry, and/or any device that manipulates signals (analog and/or digital) based on hard coding of the circuitry and/or operational instructions. Control circuitry referenced herein may further comprise one or more, storage devices, which may be embodied in a single memory device, a plurality of memory devices, and/or embedded circuitry of a device. Such data storage may comprise read-only memory, random access memory, volatile memory, non-volatile memory, static memory, dynamic memory, flash memory, cache memory, data storage registers, and/or any device that stores digital information. It should be noted that in examples in which control circuitry comprises a hardware and/or software state machine, analog circuitry, digital circuitry, and/or logic circuitry, data storage device(s)/register(s) storing any associated operational instructions may be embedded within, or external to, the circuitry comprising the state machine, analog circuitry, digital circuitry, and/or logic circuitry. The transducer(s) 32 and/or antenna(s) 38 can be considered part of the control circuitry 34.

The antenna 38 may comprise one or more coils or loops of conductive material, such as copper wire or the like. In some examples, at least a portion of the transducer 32, control circuitry 34, and/or the antenna 38 are at least partially disposed or contained within a sensor housing 36, which may comprise any type of material, and may advantageously be at least partially hermetically sealed. For example, the housing 36 may comprise glass or other rigid material, which may provide mechanical stability and/or protection for the components housed therein. In some examples, the housing 36 is at least partially flexible. For example, the housing may comprise polymer or other flexible structure/material, which may advantageously allow for folding, bending, or collapsing of the sensor 37 to allow for transportation thereof through a catheter or other introducing means.

The transducer 32 may comprise any type of sensor means or mechanism. For example, the transducer 32 may be a force-collector-type pressure sensor. In some examples, the transducer 32 comprises a diaphragm, piston, bourdon tube, bellows, or other strain- or deflection-measuring component(s) to measure strain or deflection applied over an area/surface thereof. The transducer 32 may be associated with the housing 36, such that at least a portion thereof is contained within or attached to the housing 36. With respect to sensor devices/components being "associated with" a stent or other implant structure, such terminology may refer to a sensor device or component being physically coupled, attached, or connected to, or integrated with, the implant structure.

In some examples, the transducer 32 comprises or is a component of a piezoresistive strain gauge, which may be configured to use a bonded or formed strain gauge to detect strain due to applied pressure, wherein resistance increases as pressure deforms the component/material. The transducer 32 may incorporate any type of material, including but not limited to silicon (e.g., monocrystalline), polysilicon thin film, bonded metal foil, thick film, silicon-on-sapphire, sputtered thin film, and/or the like.

In some examples, the transducer 32 comprises or is a component of a capacitive pressure sensor including a diaphragm and pressure cavity configured to form a variable capacitor to detect strain due to pressure applied to the diaphragm. The capacitance of the capacitive pressure sensor may generally decrease as pressure deforms the diaphragm. The diaphragm may comprise any material(s), including but not limited to metal, ceramic, silicon, and the like. In some examples, the transducer 32 comprises or is a component of an electromagnetic pressure sensor, which may be configured to measure the displacement of a diaphragm by means of changes in inductance, linear variable displacement transducer (LVDT) functionality, Hall Effect, or eddy current sensing. In some examples, the transducer 32 comprises or is a component of a piezoelectric strain sensor. For example, such a sensor may determine strain (e.g., pressure) on a sensing mechanism based on the piezoelectric effect in certain materials, such as quartz.

In certain examples, the monitoring system 40 can comprise at least two subsystems, including an implantable internal subsystem or device 30 that includes the sensor transducer(s) 32, as well as control circuitry 34 comprising one or more microcontroller(s), discrete electronic component(s), and one or more power and/or data transmitter(s) 38 (e.g., antennae coil). The monitoring system 40 can further include an external (e.g., nonimplantable) subsystem that includes an external reader 42 (e.g., coil), which may include a wireless transceiver that is electrically and/or communicatively coupled to certain control circuitry 41. In certain examples, both the internal 30 and external 42 subsystems include a corresponding coil antenna for wireless communication and/or power delivery (e.g., using inductive coupling) through patient tissue disposed therebetween.

The shunt structure 31 can include a percutaneously-deliverable shunt device configured to be secured to and/or in a tissue wall to provide a flow path between two chambers and/or vessels of the heart, as described in detail throughout the present disclosure. Although certain components are illustrated in Figure 8 as part of the implant device 30, it should be understood that the sensor implant device 30 may only comprise a subset of the illustrated components/modules and can comprise additional components/modules not illustrated.

The wireless signals generated by the implant device 30 can be received by the local external monitor device or subsystem 42, which can include a reader/antenna-interface circuitry module 43 configured to receive the wireless signal transmissions from the implant device 30, which is disposed at least partially within the patient 44. For example, the module 43 may include transceiver device(s)/circuitry.

The external local monitor 42 can receive the wireless signal transmissions from the implant device 30 and/or provide wireless power to the implant device 30 using an external antenna 48, such as a wand device. The reader/antenna-interface circuitry 43 can include radio-frequency (RF) (or other frequency band) front-end circuitry configured to receive and amplify the signals from the implant device 30, wherein such circuitry can include one or more filters (e.g., band-pass filters), amplifiers (e.g., low-noise amplifiers), analog-to-digital converters (ADC) and/or digital control interface circuitry, phase-locked loop (PLL) circuitry, signal mixers, or the like. The reader/antenna-interface circuitry 43 can further be configured to transmit signals over a network 49 to a remote monitor subsystem or device 46. The RF circuitry of the reader/antenna-interface circuitry 43 can further include one or more of digital-to-analog converter (DAC) circuitry, power amplifiers, low-pass filters, antenna switch modules, antennas or the like for treatment/processing of transmitted signals over the network 49 and/or for receiving signals from the implant device 30. In certain examples, the local monitor 42 includes control circuitry 41 for performing processing of the signals received from the implant device 30. The local monitor 42 can be configured to communicate with the network 49 according to a known network protocol, such as Ethernet, Wi-Fi, or the like. In certain examples, the local monitor 42 comprises a smartphone, laptop computer, or other mobile computing device, or any other type of computing device.

In certain examples, the implant device 30 includes some amount of volatile and/or non-volatile data storage. For example, such data storage can comprise solidstate memory utilizing an array of floating-gate transistors, or the like. The control circuitry 34 may utilize data storage for storing sensed data collected over a period of time, wherein the stored data can be transmitted periodically to the local monitor 42 or another external subsystem. In certain examples, the implant device 30 does not include any data storage. The control circuitry 34 may be configured to facilitate wireless transmission of data generated by the sensor transducer(s) 32, or other data associated therewith. The control circuitry 34 may further be configured to receive input from one or more external subsystems, such as from the local monitor 42, or from a remote monitor 46 over, for example, the network 49. For example, the implant device 30 may be configured to receive signals that at least partially control the operation of the implant device 30, such as by activating/deactivating one or more components or sensors, or otherwise affecting operation or performance of the implant device 30.

The one or more components of the implant device 30 can be powered by one or more power sources 35. Due to size, cost and/or electrical complexity concerns, it may be desirable for the power source 35 to be relatively minimalistic in nature. For example, high-power driving voltages and/or currents in the implant device 30 may adversely affect or interfere with operation of the heart or other body part associated with the implant device. In certain examples, the power source 35 is at least partially passive in nature, such that power can be received from an external source wirelessly by passive circuitry of the implant device 30, such as through the use of short-range, or near-field wireless power transmission, or other electromagnetic coupling mechanism. For example, the local monitor 42 may serve as an initiator that actively generates an RF field that can provide power to the implant device 30, thereby allowing the power circuitry of the implant device to take a relatively simple form factor. In certain examples, the power source 35 can be configured to harvest energy from environmental sources, such as fluid flow, motion, or the like. Additionally or alternatively, the power source 35 can comprise a battery, which can advantageously be configured to provide enough power as needed over the monitoring period (e.g., 3, 5, 10, 20, 30, 40, or 90 days, or other period of time).

In some examples, the local monitor device 42 can serve as an intermediate communication device between the implant device 30 and the remote monitor 46. The local monitor device 42 can be a dedicated external unit designed to communicate with the implant device 30. For example, the local monitor device 42 can be a wearable communication device, or other device that can be readily disposed in proximity to the patient 44 and implant device 30. The local monitor device 42 can be configured to continuously, periodically, or sporadically interrogate the implant device 30 in order to extract or request sensor-based information therefrom. In certain examples, the local monitor 42 comprises a user interface, wherein a user can utilize the interface to view sensor data, request sensor data, or otherwise interact with the local monitor system 42 and/or implant device 30.

The system 40 can include a secondary local monitor 47, which can be, for example, a desktop computer or other computing device configured to provide a monitoring station or interface for viewing and/or interacting with the monitored cardiac pressure data. In an example, the local monitor 42 can be a wearable device or other device or system configured to be disposed in close physical proximity to the patient and/or implant device 30, wherein the local monitor 42 is primarily designed to receive/transmit signals to and/or from the implant device 30 and provide such signals to the secondary local monitor 47 for viewing, processing, and/or manipulation thereof. The external local monitor system 42 can be configured to receive and/or process certain metadata from or associated with the implant device 30, such as device ID or the like, which can also be provided over the data coupling from the implant device 30.

The remote monitor subsystem 46 can be any type of computing device or collection of computing devices configured to receive, process and/or present monitor data received over the network 49 from the local monitor device 42, secondary local monitor 47, and/or implant device 30. For example, the remote monitor subsystem 46 can advantageously be operated and/or controlled by a healthcare entity, such as a hospital, doctor, or other care entity associated with the patient 44. Although certain examples disclosed herein describe communication with the remote monitor subsystem 46 from the implant device indirectly through the local monitor device 42, in certain examples, the implant device 30 can comprise a transmitter capable of communicating over the network 49 with the remote monitor subsystem 46 without the necessity of relaying information through the local monitor device 42.

As referenced above, shunt and/or other implant devices/structures may be integrated with sensor, antenna/transceiver, and/or other components to facilitate in vivo monitoring of pressure and/or other physiological parameter(s). Sensor devices in accordance with examples of the present disclosure may be integrated with cardiac shunt structures/devices or other implant devices using any suitable or desirable attachment or integration mechanism or configuration.

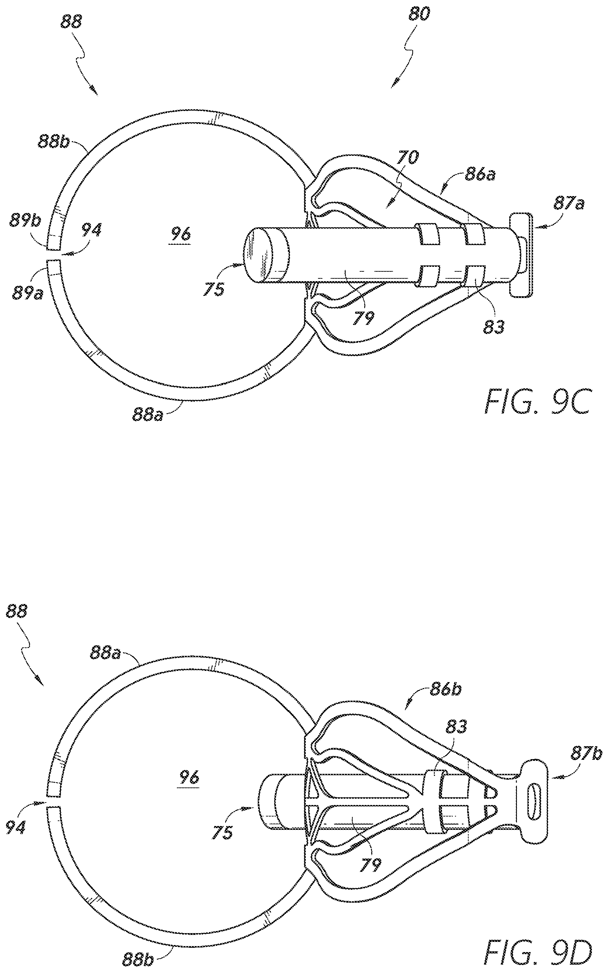

Figures 9A, 9B, 9C, and 9D illustrate perspective, side, and axial views, respectively, of a shunt sensor implant device 80 in accordance with one or more examples. Figure 10 illustrates an example sensor device/assembly 70 that may be used in sensor implant devices, such as in the sensor implant device 80 shown in Figures 9A-9D, in accordance with one or more examples of the present disclosure.

The shunt sensor implant device 80 includes two barrel-forming wings/panels 88a, 88b configured to be curved/flexed such that edges 89a, 89b thereof come into contact or into proximity (e.g., within 2 mm) with one another so as to form a tubular fluid conduit/channel. The device 80 may further include a plurality of anchor arms 86a, 86b, which may emanate from opposite axial ends/sides of the barrel 88 of the device 80. In some examples, a sensor device 70 is secured to one or more of the anchor arms 86. The sensor device 70 may be secured to the anchor arm 86 using any suitable means or mechanism. For example, securement/attachment means/mechanisms that may be suitable for attaching the sensor device 70 to any of the arms or other structure(s) of the shunt device 80 may be any of the features disclosed in PCT Application No. PCT/US20/56746, Filed on October 22, 2020, and entitled "Sensor Integration in Cardiac Implant Devices". For example, the arm(s) 86 may include one or more sensor-retention fingers, clamps, wraps, bands, belts, clips, pouches, housings, encasements, and/or the like configured to secure the sensor device 70 to an arm and/or strut or another structural feature of the device 80.

With reference to Figure 10, the sensor device/assembly 70 can include a sensor transducer component 75 and an antenna component 71. The sensor transducer component 75 may comprise any type of sensor transducer as described in detail above. In some examples, the sensor device 70 may be attached to or integrated with an arm member 86a of the shunt device 80, as shown. For example, the arm 86a with which the sensor device 70 is associated may be generally associated with a distal or proximal axial portion/end of the barrel 88. That is, when the shunt device 80 is implanted, one or more arms of the shunt 80 may be associated with an inlet/distal portion of the barrel 88, whereas one or more other anchor arms may be associated with an outlet/proximal portion of the barrel 88. Although distal and proximal sides/portions are in some contexts herein, it should be understood that identified distal portions/sides may be outlet or inlet sides of the relevant shunt structure, as with identified proximal portions/sides. Furthermore, the terms "distal" and "proximal" are used for convenience and may or may not refer to relative orientation with respect to a delivery system/device used to implant the relevant sensor implant device and/or shunt structure.

The sensor transducer component 75 includes a sensor element 77, such as a pressure sensor transducer/membrane. Relative to the arm member 86a of the shunt 80, the sensor device 70 may be attached/positioned at/on a distal 901, medial 902, and/or proximal 903 portion or area of the arm/anchor 86a, or any portion therebetween. For example, the illustrated example of Figures 9A-9D has the sensor device 70 disposed primarily on the medial area 902 and distal area 901 of the arm/anchor 86a. In some examples, readings acquired by the sensor device 70 may be used to guide titration of medication for treatment of a patient in whom the implant device 80 is implanted.

As described herein, the sensor device 70 may be configured to implement wireless data and/or power transmission. The sensor device 70 may include the antenna component 71 for such purpose. The antenna 71, as well as one or more other components of the sensor device 70, may be contained at least partially within a sensor housing 79, which may further have disposed therein certain control circuitry 72 configured to facilitate wireless data and/or power communication functionality. In some examples, the antenna component 71 comprises one or more conductive coils 73, which may facilitate inductive powering and/or data transmission. In examples comprising conductive coil(s), such coil(s) may be wrapped/disposed at least partially around a magnetic (e.g., ferrite, iron) core 89.

The sensor device 70 may be associated with either axial side/end of the shunt device 80 and/or barrel 88, wherein the different axial sides/ends of the shunt device 80 are exposed on opposite sides (*S₁, S₂*) of a tissue wall when the implant device 80 is implanted in the tissue wall.

The sensor device 70 may advantageously be biocompatible. For example, the housing 79 may advantageously be biocompatible, such as a housing comprising glass or other biocompatible material. However, at least a portion of the sensor transducer element/membrane 77, such as a diaphragm or other component, may be exposed to the external environment in some examples in order to allow for pressure readings, or other parameter sensing, to be implemented. The housing 79 may comprise an at least partially rigid cylindrical or tube-like form, such as a glass cylinder form. In some examples, the sensor transducer component 75/67 is approximately 3 mm or less in diameter. The antenna 71 may be approximately 20 mm or less in length.

The sensor device 70 may be configured to communicate with an external system when implanted in a heart or other area of a patient's body. For example, the antenna 71 may receive power wirelessly from the external system and/or communicate sensed data or waveforms to and/or from the external system. The sensor device 70 may be attached to, or integrated with, the shunt device 80 in any suitable or desirable way, such as using mechanical attachment means.

The sensor element 77 may comprise a pressure transducer. For example, the pressure transducer may be a microelectromechanical system (MEMS) transducer comprising a semiconductor diaphragm component. In some examples, the transducer may include an at least partially flexible or compressible diaphragm component, which may be made from silicone or other flexible material. The diaphragm component may be configured to be flexed or compressed in response to changes in environmental pressure. The control circuitry 72 may be configured to process signals generated in response to said flexing/compression to provide pressure readings. In some examples, the diaphragm component is associated with a biocompatible layer on the outside surface thereof, such as silicon nitride (e.g., doped silicon nitride) or the like. The diaphragm component and/or other components of the pressure transducer 77 may advantageously be fused or otherwise sealed to/with the housing 79 of the sensor device 70 in order to provide hermetic sealing of at least some of the sensor components.

The sensor retention feature(s) 83 associated with the anchor arm 94 may have any suitable or desirable form. For example, the sensor-retention feature(s) 83 may comprise one or more sensor retention fingers or other bands, straps, wraps, coils, wires, adhesives, clamps, clips, apertures, engagement projections or forms, locks, or other retention features. In some examples, the anchor arm 86a includes a distal stopper feature 87a, such as a tab or similar form/structure, configured to limit distal movement of the sensor device 70 beyond the distal end of the shunt arm 86a. For example, the stopper feature 87a may be a tab that is folded to cover the radial profile of the sensor device 70 in a manner as to restrict axial movement in at least one direction of the sensor device 70. In some examples, the sensor device 70 is integrated with the arm 86a, such that separate retention features are not necessary to secure the sensor device 70 to the shunt device 80. For example, the anchor arm 86a may be integral with the housing 79 of the sensor device 70. As with any example of a shunt device disclosed herein, the barrel/conduit 88 that defines the shunt orifice may be covered internally and/or externally, at least in part, with fabric or other covering, which may provide sealing for the device.

The sensor device 70 may advantageously be disposed, position, secured, oriented, and/or otherwise situated in a configuration in which the sensor transducer component 75 thereof is disposed within a channel area of the shunt device 80. The term "channel area" is used herein according to its broad and ordinary meaning and may refer to a three-dimensional space defined by a radial boundary of a fluid conduit and extending from the fluid conduit about an axis of the fluid conduit.

Figure 9C illustrates an axial view of the implant device 80 of Figures 9A and 9B in accordance with one or more examples of the present disclosure. Specifically, Figure 9C shows an axial view that corresponds to an axial side of the implant device 80 associated with the sensor device 70. That is, the sensor component 75 is attached to, integrated with, or otherwise associated with the arm 86a, the side of which is shown facing out of the page in Figure 9C. The side shown facing out of the page in Figure 9C may be a distal or proximal side.

The sensor-retention feature(s) 83 may circumferentially encase or retain the sensor device 70, or a portion thereof. In some examples, the sensor device 70 may be attached to the arm 86a through the application of mechanical force, either through sliding the sensor 70 through certain retention features 83 or through clipping, locking, or otherwise engaging the sensor 70 with the arm 86a by pressing or applying other mechanical force thereto. In some examples, the shunt device 80 may comprise one or more tabs that may be configured to pop-up or extend on one or more sides of the sensor device 70 for mechanical fastening. Such tabs may comprise memory metal (e.g., nitinol) or other at least partially rigid material. In some examples, the sensor device 70 is pre-attached to the arm 86a and/or integrated therewith prior to implantation. In some examples, the sensor 70 may be built or manufactured into the shunt device 80 to form a unitary structure. For example, in some examples, the sensor 70 may be attached to or integrated with the arm member 86a of the shunt device 80.

Figure 9D illustrates another axial view of the implant device 80 of Figures 9A and 9B in accordance with one or more examples of the present disclosure. Specifically, Figure 9D shows an axial view that corresponds to an axial side of the implant device 80 that is opposite the sensor device 70. The side shown facing out of the page in Figure 9D may be a distal or proximal side.

As mentioned above, a sensor device may be secured to a shunt implant device of the present disclosure and/or anchor arm thereof using any suitable or desirable sensor-retention means. Figure 11-1 shows a sensor implant device 120 having a suturewrapped sensor device 126 associated therewith in accordance with one or more examples. The device 126 includes one or more wraps of suture 128 (e.g., PET stitching, or cloth strip(s) or the like) configured to at least partially secure the sensor device 126 to the anchor arm 124. In some examples, the wrap 128 is wrapped in strands circumferentially and/or axially on the sensor cylinder and around the anchor arm 124. The suture wrap 128 can may be wrapped around the cylinder/sensor 126 in a circumferential fashion traversing at least a portion of the length of the sensor 126. In some examples, the suture wrap 128 has a sheetlike cover/wrap pulled or applied over the sensor 126 and/or the anchor arm 124. For example, sutures or other type of line or stitching may be wrapped around the cover/wrap to secure the cover/wrap to the sensor 126 and the arm 124. The suture(s)/line 128 may comprise ePTFE, PET, or the like. It may be desirable to protect suture features from tissue in-growth using an appropriate coating, covering, or similar.

Figure 11-2 shows a sensor implant device 130 having a sensor-retention pouch 138 in accordance with one or more examples. The pouch 130 may comprise a membrane sock- or wrap-type retention means or feature configured to at least partially secure the sensor implant device 130 to the sensor-support strut/arm. The membrane pouch/wrap can comprise polytetrafluoroethylene (PTFE) and/or polyurethane (PU) (e.g., electrospun or rotary-jet-spun) membrane. The pouch or sock 138 can be attached to or otherwise associated with an anchor arm 134 or another portion of the shunt structure. For example, the pouch 138 may be a suture-based or cloth-based (e.g., fibrous and/or polymer cloth) pouch, wrapping, or other retention material and/or form. The pouch 138 can comprise any suitable or desirable material, including polytetrafluoroethylene (PTFE), expanded polytetrafluoroethylene (ePTFE), polyurethane (PU), or the like and/or combinations of similar materials. Such material may be electrospun onto the sensor 136 in some implementations, or may be applied using rotary jet spinning.

In some examples, the sensor 136 is configured to be slidingly disposed within the pouch 138, wherein tension and/or compression of the pouch 138 serves to retain the sensor 136 in a fixed position within the pouch 138. Although a pouch/wrap is illustrated in Figure 11-2 that envelops at least a portion of the sensor 136 in a sock-/tube-like manner, in some examples, the pouch 138 comprises a band or other non-enveloping retention means. In some examples, the sensor 136 may be sutured or otherwise attached or fixed to the pouch 138. Furthermore, the pouch 138 may be sutured or otherwise fixed or attached to the arm member 134 of the shunt structure 139. The pouch 138 may advantageously be open on one or both axial ends thereof to allow for fluid contact with the sensor element/transducer 137 associated with the sensor 136. That is, the sensor 136 may be exposed through an open portion on a distal or proximal end of the arm 134 and/or pouch 138.

Although certain examples are disclosed herein in the context of sensor implant devices including a single sensor device associated with a shunt structure, it should be understood that shunt sensor implant devices in accordance with aspects of the present disclosure may have any suitable or desirable number of sensor devices associated therewith.

Figure 11-3 shows a sensor implant device 140 having a sensor-retention cup 148 in accordance with one or more examples. The cup 148 may comprise an over-mold support form. A sensor 146 is nestingly disposed at least partially within the cup form 148. The cup 148 may be rigid or flexible. In some examples, the cup 148 is bonded to the sensor 146 and/or anchor arm 144 through heat setting or other process. The sensor 146 may be inserted into the cup form 148, or the cup 148 may be applied over the sensor 146 and the anchor arm 144 after placement of the sensor 146 on the anchor arm 144. A polymer wrap may be applied over the cup 148 and sensor 146 to further secure the sensor 146 within the cup 148.

Figure 12 shows a shunt implant device 80 implanted in a coronary sinus tissue wall 21 in accordance with one or more examples. Although the shunt implant device 80 is shown as including a sensor 70, it should be understood that the shunt device 80 may or may not incorporate sensor component(s)/functionality. With respect to sensor-equipped examples, the implant device 80 may be implanted in a configuration such that the sensor transducer component 755 is at least partially exposed on the atrial side of the tissue wall 21, as shown.

The coronary sinus 16 is generally contiguous around the left atrium 2, and therefore there are a variety of possible acceptable placements for the implant device 80. The target site selected for placement of the implant device 80 may be made in an area where the tissue of the particular patient is less thick or less dense, as determined beforehand by noninvasive diagnostic means, such as a CT scan or radiographic technique, such as fluoroscopy or intravascular coronary echo (IVUS). With the sensor transducer component 75 disposed in the channel area of the shunt conduit 88, the sensor transducer 65 may advantageously be disposed in an area of flow that is relatively high, thereby allowing for sensor readings to be generated indicating characteristics of the flow through the conduit 88 of the shunt structure 80.

In some cases, left-to-right shunting through implantation of the shunt device 80 in the wall 21 between the left atrium 2 and the coronary sinus 16 can be preferable to shunting through the atrial septum, which is shown in Figure 13. For example, shunting through the coronary sinus 16 can provide reduced risk of thrombus and embolism. The coronary sinus is less likely to have thrombus/emboli present for several reasons. First, the blood draining from the coronary vasculature into the right atrium 5 has just passed through capillaries, so it is essentially filtered blood. Second, the ostium 14 of the coronary sinus in the right atrium is often partially covered by a pseudo-valve called the Thebesian Valve (not shown). The Thebesian Valve is not always present, but some studies show it is present in most hearts and can block thrombus or other emboli from entering in the event of a spike in right atrium pressure. Third, the pressure gradient between the coronary sinus and the right atrium into which it drains is generally relatively low, such that thrombus or other emboli in the right atrium is likely to remain there. Fourth, in the event that thrombus/emboli do enter the coronary sinus, there will be a much greater gradient between the right atrium and the coronary vasculature than between the right atrium and the left atrium. Most likely, thrombus/emboli would travel further down the coronary vasculature until right atrium pressure returned to normal and then the emboli would return directly to the right atrium.

Some additional advantages to locating the shunt structure 80 between the left atrium 2 and the coronary sinus 16 is that this anatomy is generally more stable than the interatrial septal tissue. By diverting left atrial blood into the coronary sinus, sinus pressures may increase by a small amount. This can cause blood in the coronary vasculature to travel more slowly through the heart, increasing perfusion and oxygen transfer, which can be more efficient and also can help a dying heart muscle to recover. In addition, by implanting the shunt device/structure 80 in the wall of the coronary sinus 16, damage to the atrial septum may be prevented. Therefore, the atrial septum may be preserved for later transseptal access for alternate therapies.

Figure 13 shows a shunt implant device/structure 80 implanted in an atrial septum 18 in accordance with one or more examples. The particular position in the atrial septum wall 18 may be selected or determined to provide a relatively secure anchor location for the shunt implant device 80. Furthermore, the shunt device/structure 80 may be implanted at a position that is desirable in consideration of future re-crossing of the septal wall 18 for future interventions. Implantation of the shunt device/structure 73 in the atrial septum wall 18 may advantageously allow for fluid communication between the left 2 and right 5 atria.

Although the shunt implant device 80 is shown as including a sensor 70, it should be understood that the shunt device 80 may or may not incorporate sensor component(s)/functionality. With respect to sensor-equipped examples, the implant device 80 may be implanted in a configuration such that a sensor 70 of the device 80 is exposed in the left atrium 2. Alternatively, the sensor implant device 80 may be implanted in a configuration such that the sensor 70 is exposed in the right atrium 2.

Figures 14-1, 14-2, 14-3, 14-4, and 14-5 provide a flow diagram illustrating a process 1400 for implanting an adjustable-barrel shunt implant device in accordance with one or more examples. Figures 15-1, 15-2, 15-3, 15-4, and 15-5 provide images of cardiac anatomy and certain devices/systems corresponding to operations of the process 1400 of Figures 14-1, 14-2, 14-3, 14-4, and 14-5 in accordance with one or more examples.

At block 1402, the process 1400 involves providing a delivery system 151 with a shunt implant device 80 disposed therein in a delivery configuration, such as a sensor-equipped shunt implant device as disclosed in detail herein. Image 1502 of Figure 15-1 shows a partial cross-sectional view of the delivery system 151 for the shunt implant device 80 in accordance with one or more examples of the present disclosure. The image 1502 shows the shunt implant device 80 disposed within an outer sheath 150 of the delivery system 151. Although a particular example of a delivery system is shown in Figure 15-1, it should be understood that adjustable-barrel shunt implant devices in accordance with aspects of the present disclosure may be delivered and/or implanted using any suitable or desirable delivery system and/or delivery system components.

The illustrated delivery system 151 includes an inner catheter 155, which may be disposed at least partially within the outer sheath 150 during one or more portions of the process 1400. In some examples, the shunt structure of the implant device 80 may be disposed at least partially around the inner catheter 155, wherein the shunt structure is disposed at least partially within the outer sheath 150 during one or more portions of the process 1400. For example, the inner catheter 155 may be disposed within the barrel portion 88 of the shunt 80, as shown.

In some examples, the delivery system 151 may be configured such that a guidewire 153 may be disposed at least partially therein. For example, the guidewire 153 may run in the area of an axis of the sheath 150 and/or inner catheter 155, such as within the inner catheter 155, as shown. The delivery system 151 may be configured to be advanced over the guidewire 153 to guide the delivery system 151 to a target implantation site.

In some examples, the delivery system 51 includes a tapered nosecone feature 152, which may be associated with a distal end of the sheath 150, the catheter 155, and/or the delivery system 51. In some implementations, the nosecone feature 152 may be utilized to dilate the opening in a tissue wall into which the shunt implant device 80 is to be implanted, or through which the delivery system is to be advanced. The nosecone feature 152 may facilitate advancement of the distal end of the delivery system 151 through the tortuous anatomy of the patient and/or within an outer delivery sheath or other conduit/path. The nosecone 152 may be a separate component from the catheter 155 or may be integrated with the catheter 155. In some examples, the nosecone 152 is adjacent to and/or integrated with a distal end of the catheter 155. In some examples, the nosecone 152 may comprise and/or be formed of multiple flap-type forms that can be urged/spread apart when the shunt implant device 80 and/or any portions thereof, the interior catheter 155, or other device(s) are advanced therethrough.

In some examples, the shunt implant device 80 may be disposed in the delivery system 151 with a sensor device 70, as described in detail herein, attached thereto or otherwise associated therewith. In some examples, the inner catheter 155 includes one or more cut-outs, indentations, recesses, gaps, openings, apertures, holes, slits, or other features configured to accommodate the presence of the sensor device 70 and/or other feature(s) or aspect(s) of the implant device 80. For example, the sensor device 70 may be disposed at least partially within an inner diameter of the shunt structure 80 in the radially-compressed delivery configuration shown in Figure 15-1. In such configurations, the sensor assembly component(s) may create an interference with respect to the ability of the shunt structure 80 to be disposed relatively tightly around the inner catheter 155, thereby potentially increasing the profile of the delivery system and/or affecting the ability of the implant device 80 to be delivered using the delivery system 151. Therefore, as shown in Figure 15-1, the inner catheter 155 may include one or more sensor device accommodation features, such as a sensor cut-out or other accommodation feature 157. In some examples, the accommodation feature 157 may comprise a longitudinal and circumferential cut-out of the inner catheter 155. The accommodation feature 157 may advantageously be dimensioned to correspond to the size and/or profile of the sensor device 70, as shown, and may allow for the sensor device 70 to radially project into an inner diameter/space of the inner catheter 155.

The shunt implant device 80 can be positioned within the delivery system 151 with a first end thereof (i.e., distal anchor arm(s) 86a) disposed distally with respect to the barrel 88 of the shunt structure 80. A second end (i.e., proximal anchor arm(s) 86b) is positioned at least partially proximally with respect to the barrel 88 of the shunt 80 and/or the sensor device 70.

The outer sheath 150 may be used to transport the shunt implant device 80 to the target implantation site. That is, the shunt implant device 80 may be advanced to the target implantation site at least partially within a lumen of the outer sheath 50, such that the sensor implant device 70 is held and/or secured at least partially within a distal portion of the outer sheath 50.

As described in detail herein, the barrel 88 of the shunt implant 80 can comprise two separate barrel-forming wings/panels 88a, 88b. In the delivery configuration shown in Figure 15-1, at least one of the barrel wings/panels 88a, 88b may be inwardly- and/or radially-deflected with respect to an axis of the barrel 88, such that the wings/panels 88a, 88b circumferentially overlap one another, at least in part. The overlapping of the barrel wings/panels 88a, 88b can reduce the diameter/profile of the shunt 80 to accommodate placement in the outer sheath 150, which may have a diameter that is less than the expanded diameter of the barrel 80 (see Figure 15-5). Therefore, the adjustability/collapsibility of the barrel 88, as enabled by the implementation of the barrel wings/panels 88a, 88b, can advantageously allow for transportation/delivery of the shunt device 80 using relatively/desirably compact instrumentation/systems. Image 1501 shows an alternative design with an angled/canted barrel, as described in detail above.

At block 1404, the process 1400 involves accessing the right atrium 5 of the heart of a patient using the delivery system 151 with the shunt implant device 80 disposed therein. In some implementations, accessing the cardiac anatomy with the delivery system 151 may be performed following one or more procedures or steps to place the guidewire 53 and/or form and/or dilate an opening between the left atrium 2 and coronary sinus 16 of the patient's heart, the details of which are omitted for convenience and clarity.

Image 1504 shows various example catheter-/sheath-type delivery systems 111 that may be used to implant shunt devices in accordance with aspects of the present disclosure. The delivery systems 111 can represent examples of the delivery system 151 of image 1502. The delivery systems 111 can be steerable and relatively small in cross-sectional profile to allow for traversal of the various blood vessels and chambers through which they may be advanced en route to, for example, the right atrium 5, coronary sinus 16, left atrium 2 or other anatomy or chamber. Catheter access to the right atrium 5, coronary sinus 16, or left atrium 2 in accordance with certain transcatheter solutions may be made via the inferior vena cava 29 (as shown by the catheter 111a) or the superior vena cava 19 (as shown by the catheter 111b).

Although access to the right and/or left atria is illustrated and described in connection with certain examples as being via the right atrium and/or vena cavae, such as through a transfemoral or other transcatheter procedure, other access paths/methods may be implemented in accordance with examples of the present disclosure. For example, in cases in which septal crossing through the interatrial septal wall is not possible, other access routes may be taken to the left atrium 2. In patients suffering from a weakened and/or damaged atrial septum, further engagement with the septal wall can be undesirable and result in further damage to the patient. Furthermore, in some patients, the septal wall may be occupied with one or more implant devices or other treatments, wherein it is not tenable to traverse the septal wall in view of such treatment(s). As alternatives to transseptal access, transaortic access may be implemented, wherein a delivery catheter is passed through the descending aorta 32, aortic arch 12, ascending aorta, and aortic valve 7, and into the left atrium 2 through the mitral valve 6. Alternatively, transapical access may be implemented to access the target anatomy through the apex of the heart, such as using a minimally-invasive access through the chest wall.

At block 1406, the process 1400 involves advancing the delivery system 151 into the coronary sinus 16 to a target implantation site adjacent a wall 21 separating the coronary sinus 16 from the left atrium 2. Access to the target wall 21 and left atrium 2 via the coronary sinus 16 may be achieved using any suitable or desirable procedure.

At block 1408, the process 1400 involves accessing the left atrium 2 through an opening 99 formed in the wall 21. For example, the guidewire 153 may be disposed as running through the opening 99 prior to penetration thereof by the nosecone 152. The opening 99 may originally be formed using a needle (not shown) associated with the delivery system 151 or other delivery system implemented prior to block 1408. In some implementations, the nosecone feature 152 may be used to at least partially dilate the opening 99, which may have been previously dilated using a balloon dilator or other instrument.

At block 1410, the process 1400 involves deploying one or more anchor arms 86a, which may be considered the distal anchor arm(s) of the shunt implant device 80, on the atrial side of the wall 21. The distal arm 86a can have associated therewith the sensor device 70, such that a sensor transducer 75 of the sensor device 70 is exposed within the left atrium 2, wherein the sensor transducer 75 can be used to obtain signals indicating physiological parameters associated with the left atrium, such as pressure.

At block 1412, the process 1400 involves deploying one or more proximal arms 86b of the shunt implant device 80 on a coronary sinus side of the tissue wall 21 to thereby sandwich portion(s) of the wall 21 between the distal and proximal arms 86 of the shunt structure 80. Once the barrel 88 is positioned in the opening 99, a balloon catheter or other device may be used to expand the barrel wings/panels 88a, 88b to the expanded-barrel configuration shown in image 1513 of Figure 15-5. In some implementations, the expansion of the barrel wings/panels 88a, 88b may be effected through shape memory of the shunt implant 80, which may be pre-shaped to the expanded configuration, such that release of the barrel 88 from the delivery system 151 causes the barrel wings/panels 88a, 88b to automatically expand to the expanded configuration shown in image 1513. Once the barrel wings/panels 88a, 88b have expanded (e.g., through outward/radial deflection) to a point that they no longer overlap, the wings/panels may occupy common radial area, such that mechanical interference between distal edges 89a, 89b of the wings/panels prevents collapsing/compression thereof after deployment/expansion, which can advantageously maintain the desired flow channel through the barrel 88 postoperatively.

At block 1414, the process 1400 involves withdrawing the delivery system 151, leaving the shunt implant device 80 implanted in the tissue wall 21, thereby allowing blood flow to be shunted through the implant device 70 from the left atrium 2 into the right side of the heart via the coronary sinus 16.

Additional aspects and features of processes for delivering shunt structures that may be integrated with sensor devices/functionality in accordance with examples of the present disclosure for implantation in the wall between the coronary sinus and the left atrium are disclosed in U.S. Pat. No. 9,789,294, entitled "Expandable Cardiac Shunt," issued on October 15, 2017. Although the implant device 80 is shown in the left atrium/coronary sinus wall 21, the implant device 80 may be positioned between other cardiac chambers, such as between the left and right atria. intended to imply that features, elements and/or steps are in any way required for one or more examples or that one or more examples necessarily include logic for deciding, with or without author input or prompting, whether these features, elements and/or steps are included or are to be performed in any particular example. The terms "comprising," "including," "having," and the like are synonymous, are used in their ordinary sense, and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y and Z," unless specifically stated otherwise, is understood with the context as used in general to convey that an item, term, element, etc. may be either X, Y or Z. Thus, such conjunctive language is not generally intended to imply that certain examples require at least one of X, at least one of Y and at least one of Z to each be present.

It should be appreciated that in the above description of examples, various features are sometimes grouped together in a single example, Figure, or description thereof for the purpose of streamlining the disclosure and aiding in the understanding of one or more of the various inventive aspects. This method of disclosure, however, is not to be interpreted as reflecting an intention that any claim require more features than are expressly recited in that claim. Moreover, any components, features, or steps illustrated and/or described in a particular example herein can be applied to or used with any other example(s). Further, no component, feature, step, or group of components, features, or steps are necessary or indispensable for each example. Thus, it is intended that the scope of the inventions herein disclosed and claimed below should not be limited by the particular examples described above, but should be determined only by a fair reading of the claims that follow.

It should be understood that certain ordinal terms (e.g., "first" or "second") may be provided for ease of reference and do not necessarily imply physical characteristics or ordering. Therefore, as used herein, an ordinal term (e.g., "first," "second," "third," etc.) used to modify an element, such as a structure, a component, an operation, etc., does not necessarily indicate priority or order of the element with respect to any other element, but rather may generally distinguish the element from another element having a similar or identical name (but for use of the ordinal term). In addition, as used herein, indefinite articles ("a" and "an") may indicate "one or more" rather than "one." Further, an operation performed "based on" a condition or event may also be performed based on one or more other conditions or events not explicitly recited.

Unless otherwise defined, all terms (including technical and scientific terms) used herein have the same meaning as commonly understood by one of ordinary skill in the art to which examples belong. It be further understood that terms, such as those defined in commonly used dictionaries, should be interpreted as having a meaning that is consistent with their meaning in the context of the relevant art and not be interpreted in an idealized or overly formal sense unless expressly so defined herein.

The spatially relative terms "outer," "inner," "upper," "lower," "below," "above," "vertical," "horizontal," and similar terms, may be used herein for ease of description to describe the relations between one element or component and another element or component as illustrated in the drawings. It be understood that the spatially relative terms are intended to encompass different orientations of the device in use or operation, in addition to the orientation depicted in the drawings. For example, in the case where a device shown in the drawing is turned over, the device positioned "below" or "beneath" another device may be placed "above" another device. Accordingly, the illustrative term "below" may include both the lower and upper positions. The device may also be oriented in the other direction, and thus the spatially relative terms may be interpreted differently depending on the orientations.

Unless otherwise expressly stated, comparative and/or quantitative terms, such as "less," "more," "greater," and the like, are intended to encompass the concepts of equality. For example, "less" can mean not only "less" in the strictest mathematical sense, but also, "less than or equal to."

## Claims

1. A shunt implant device (50; 50a; 50b, 50c; 60) comprising:
a distal and a proximal anchor arm (56; 56a; 56b; 66);
a backbone support structure (52, 62) extending axially between the distal and the proximal anchor arm (56; 56a; 56b; 66); and
first and second barrel wings (58a; 58b; 401a; 401b; 401c; 68a; 68b) emanating from the backbone support structure (52; 62) and configured to curve to form a tubular barrel form (58; 68) defining a central flow channel (96) having a first axis (A1); wherein the distal and the proximal anchor arms (56; 56a; 56b; 66) are configured to curl outward from the backbone support structure (52; 62) to point radially away from the first axis (A1) of the central flow channel (96), the tubular barrel form (56; 68) includes an axial gap (94) formed between distal edges of the first and second barrel wings (58a; 58b; 401a; 401b; 401c; 68a; 68b) and wherein the backbone support structure extends axially between the distal and proximal anchor arms on each side of the tubular barrel form.

2. The shunt implant device (50; 50a; 50b, 50c; 60) of claim 1, wherein the first and second barrel wings (58a; 58b; 401a; 401b; 401c; 68a; 68b) are configured to be inwardly deflected such that distal portions of the first and second barrel wings (58a; 58b; 401a; 401b; 401c; 68a; 68b) are circumferentially overlapped.

3. The shunt implant device (50; 50a; 50b, 50c; 60) of claim 1, wherein distal edges of the first and second barrel wings (58a; 58b; 401a; 401b; 401c; 68a; 68b) are curved towards one another and disposed proximate to one another when the first and second barrel wings (58a; 58b; 401a; 401b; 401c; 68a; 68b) form the tubular barrel form (58; 68).

4. The shunt implant device (50; 50a; 50b, 50c; 60) of any of claims 1-3, wherein, in a flattened configuration the distal and proximal anchor arms (56; 56a; 56b; 66) project in a first dimension, and the first and second barrel wings (58a; 58b; 401a; 401b; 401c; 68a; 68b) project in a second dimension that is perpendicular to the first dimension.

5. The shunt implant device (50; 50a; 50b, 50c; 60) of any of claims 1-4, wherein the barrel wings (58a; 58b; 401a; 401b; 401c; 68a; 68b) each comprise a plurality of lateral struts (417; 61) separated by one or more lateral slit gaps (92).

6. The shunt implant device (50; 50a; 50b, 50c; 60) of any of claims 1-4, wherein the barrel wings (58a; 58b; 401a; 401b; 401c; 68a; 68b) each comprise a plurality of vertical struts (441; 541) separated by one or more vertical slit gaps (411; 511).

7. The shunt implant device (50; 50a; 50b, 50c; 60) of any of claims 1-4, wherein the barrel wings (58a; 58b; 401a; 401b; 401c; 68a; 68b) comprise struts forming one or more rows of polygonal cells.

8. The shunt implant device (50; 50a; 50b, 50c; 60) of any of claims 1-4, wherein the tubular barrel form (58; 68) has an axis that is angled relative to a tissue plane associated with the shunt implant device (50; 50a; 50b, 50c; 60).

9. The shunt implant device (50; 50a; 50b, 50c; 60) of any of claims 1-8, further comprising a pressure sensor device (70) attached to any of the arms.

10. The shunt implant device (50; 50a; 50b, 50c; 60) of claim 9, wherein at least one of the proximal and distal anchor arms (56; 56a; 56b; 66) comprises a sensor-retention means (83) configured to secure the sensor device (70) to the shunt implant device (50; 50a; 50b, 50c; 60).

11. The shunt implant device (50; 50a; 50b, 50c; 60) of any of claims 1-10, wherein the backbone support structure (52; 62) comprises at least one axial strut.

12. The shunt implant device (50; 50a; 50b, 50c; 60) of claim 1, wherein the first and second barrel wings (58a; 58b; 401a; 401b; 401c; 68a; 68b) are first and second arcuate panels, wherein, in an expanded configuration, the first and second arcuate panels are radially aligned.

13. The shunt implant device (50; 50a; 50b, 50c; 60) of claim 12, wherein contact between edges of the first and second arcuate panels prevents radial compression of the tubular barrel form (58; 68).

14. The shunt implant device (50; 50a; 50b, 50c; 60) of any of claims 1-13, wherein the first and second barrel wings (58a; 58b; 401a; 401b; 401c; 68a; 68b) are configured to assume a compressed delivery configuration in which a first one of the first and second barrel wings (58a; 58b; 401a; 401b; 401c; 68a; 68b) is deflected radially inside of, and circumferentially overlapping, a second one of the first and second barrel wings (58a; 58b; 401a; 401b; 401c; 68a; 68b).

15. The shunt implant device (50; 50a, 50b; 50c, 60) of claim 14, wherein, in the compressed delivery configuration, the tubular barrel form (58; 68) has a diameter that is less than a diameter of the tubular barrel form (58; 68) in an expanded configuration in which the first and second barrel wings (58a; 58b; 401a; 401b; 401c; 68a; 68b) are radially aligned.

## Patentansprüche

1. Shunt-Implantatvorrichtung (50; 50a; 50b, 50c; 60), umfassend:
einen distalen und einen proximalen Ankerarm (56; 56a; 56b; 66);
eine Rückgrat-Stützstruktur (52, 62), die sich axial zwischen dem distalen und dem proximalen Ankerarm (56; 56a; 56b; 66) erstreckt; und
einen ersten und einen zweiten Trommelflügel (58a; 58b; 401a; 401b; 401c; 68a; 68b), die von der Rückgrat-Stützstruktur (52; 62) ausgehen und dazu konfiguriert sind, sich zu krümmen, um eine röhrenförmige Trommelform (58; 68) zu bilden, die einen zentralen Strömungskanal (96) mit einer ersten Achse (A1) definiert;
wobei der distale und der proximale Ankerarm (56; 56a; 56b; 66) dazu konfiguriert sind, sich von der Rückgrat-Stützstruktur (52; 62) nach außen einzurollen, um von der ersten Achse (A1) des zentralen Strömungskanals (96) radial weg zu weisen, wobei die röhrenförmige Trommelform (56; 68) einen axialen Spalt (94) enthält, der zwischen distalen Kanten des ersten und des zweiten Trommelflügels (58a; 58b; 401a; 401b; 401c; 68a; 68b) gebildet ist, und wobei sich die Rückgrat-Stützstruktur zwischen dem distalen und dem proximalen Ankerarm auf jeder Seite der röhrenförmigen Trommelform axial erstreckt.

2. Shunt-Implantatvorrichtung (50; 50a; 50b, 50c; 60) nach Anspruch 1, wobei der erste und der zweite Trommelflügel (58a; 58b; 401a; 401b; 401c; 68a; 68b) dazu konfiguriert sind, nach innen ausgelenkt zu werden, so dass distale Abschnitte des ersten und des zweiten Trommelflügels (58a; 58b; 401a; 401b; 401c; 68a; 68b) in Umfangsrichtung überlappen.

3. Shunt-Implantatvorrichtung (50; 50a; 50b, 50c; 60) nach Anspruch 1, wobei distale Kanten des ersten und des zweiten Trommelflügels (58a; 58b; 401a; 401b; 401c; 68a; 68b) zueinander gekrümmt und nahe beieinander angeordnet sind, wenn der erste und der zweite Trommelflügel (58a; 58b; 401a; 401b; 401c; 68a; 68b) die röhrenförmige Trommelform (58; 68) bilden.

4. Shunt-Implantatvorrichtung (50; 50a; 50b, 50c; 60) nach einem der Ansprüche 1 bis 3, wobei in einer abgeflachten Konfiguration der distale und der proximale Ankerarm (56; 56a; 56b; 66) in einer ersten Dimension hervorstehen und der erste und der zweite Trommelflügel (58a; 58b; 401a; 401b; 401c; 68a; 68b) in einer zweiten Dimension hervorstehen, die senkrecht zur ersten Dimension ist.

5. Shunt-Implantatvorrichtung (50; 50a; 50b, 50c; 60) nach einem der Ansprüche 1 bis 4, wobei die Trommelflügel (58a; 58b; 401a; 401b; 401c; 68a; 68b) jeweils mehrere laterale Streben (417; 61) umfassen, die durch einen oder mehrere laterale Schlitzspalte (92) getrennt sind.

6. Shunt-Implantatvorrichtung (50; 50a; 50b, 50c; 60) nach einem der Ansprüche 1 bis 4, wobei die Trommelflügel (58a; 58b; 401a; 401b; 401c; 68a; 68b) jeweils mehrere vertikale Streben (441; 541) umfassen, die durch einen oder mehrere vertikale Schlitzspalte (411; 511) getrennt sind.

7. Shunt-Implantatvorrichtung (50; 50a; 50b, 50c; 60) nach einem der Ansprüche 1 bis 4, wobei die Trommelflügel (58a; 58b; 401a; 401b; 401c; 68a; 68b) Streben umfassen, die eine oder mehrere Reihen von polygonalen Zellen bilden.

8. Shunt-Implantatvorrichtung (50; 50a; 50b, 50c; 60) nach einem der Ansprüche 1 bis 4, wobei die röhrenförmige Trommelform (58; 68) eine Achse aufweist, die relativ zu einer der Shunt-Implantatvorrichtung (50; 50a; 50b, 50c; 60) zugeordneten Gewebeebene angewinkelt ist.

9. Shunt-Implantatvorrichtung (50; 50a; 50b, 50c; 60) nach einem der Ansprüche 1 bis 8, ferner eine Drucksensorvorrichtung (70) umfassend, die an einem der Arme angebracht ist.

10. Shunt-Implantatvorrichtung (50; 50a; 50b, 50c; 60) nach Anspruch 9, wobei wenigstens einer von dem proximalen und dem distalen Ankerarm (56; 56a; 56b; 66) ein Sensorrückhaltemittel (83) umfasst, das dazu konfiguriert ist, die Sensorvorrichtung (70) an der Shunt-Implantatvorrichtung (50; 50a; 50b, 50c; 60) zu befestigen.

11. Shunt-Implantatvorrichtung (50; 50a; 50b, 50c; 60) nach einem der Ansprüche 1 bis 10, wobei die Rückgrat-Stützstruktur (52; 62) wenigstens eine axiale Strebe umfasst.

12. Shunt-Implantatvorrichtung (50; 50a; 50b, 50c; 60) nach Anspruch 1, wobei der erste und der zweite Trommelflügel (58a; 58b; 401a; 401b; 401c; 68a; 68b) ein erstes und ein zweites bogenförmiges Panel sind, wobei in einer expandierten Konfiguration das erste und das zweite bogenförmige Panel radial ausgerichtet sind.

13. Shunt-Implantatvorrichtung (50; 50a; 50b, 50c; 60) nach Anspruch 12, wobei ein Kontakt zwischen Kanten des ersten und des zweiten bogenförmigen Paneels eine radiale Kompression der röhrenförmigen Trommelform (58; 68) verhindert.

14. Shunt-Implantatvorrichtung (50; 50a; 50b, 50c; 60) nach einem der Ansprüche 1 bis 13, wobei der erste und der zweite Trommelflügel (58a; 58b; 401a; 401b; 401c; 68a; 68b) dazu konfiguriert sind, eine komprimierte Zuführungskonfiguration anzunehmen, in welcher ein erster von dem ersten und dem zweiten Trommelflügel (58a; 58b; 401a; 401b; 401c; 68a; 68b) radial innerhalb von einem zweiten von dem ersten und dem zweiten Trommelflügel (58a; 58b; 401a; 401b; 401c; 68a; 68b) ausgelenkt ist und diesen in Umfangsrichtung überlappt.

15. Shunt-Implantatvorrichtung (50; 50a, 50b; 50c, 60) nach Anspruch 14, wobei in der komprimierten Zuführungskonfiguration die röhrenförmige Trommelform (58; 68) einen Durchmesser aufweist, der kleiner ist als ein Durchmesser der röhrenförmigen Trommelform (58; 68) in einer expandierten Konfiguration, in der der erste und der zweite Trommelflügel (58a; 58b; 401a; 401b; 401c; 68a; 68b) radial ausgerichtet sind.

## Revendications

1. Dispositif de shunt implantable (50 ; 50a ; 50b, 50c ; 60) comprenant :
un bras d'ancrage distal et un bras d'ancrage proximal (56 ; 56a ; 56b ; 66) ;
une structure d'ossature support (52, 62) s'étendant axialement entre le bras d'ancrage distal et le bras d'ancrage proximal (56 ; 56a ; 56b ; 66) ; et
des première et seconde ailes (58a ; 58b ; 401a ; 401b ; 401c ; 68a ; 68b) de cylindre partant de la structure d'ossature support (52 ; 62) et conçues pour s'incurver afin de former une forme cylindrique tubulaire (58 ; 68) définissant un canal d'écoulement central (96) présentant un premier axe (A1) ; dans lequel les bras d'ancrage distal et proximal (56 ; 56a ; 56b ; 66) sont conçus pour s'enrouler vers l'extérieur à partir de la structure d'ossature support (52 ; 62) de manière à pointer radialement à l'opposé du premier axe (A1) du canal d'écoulement central (96), la forme cylindrique tubulaire (56 ; 68) comporte un interstice axial (94) formé entre des bords distaux des première et seconde ailes (58a ; 58b ; 401a ; 401b ; 401c ; 68a ; 68b) de cylindre et dans lequel la structure d'ossature support s'étend axialement entre les bras d'ancrage distal et proximal de chaque côté de la forme cylindrique tubulaire.

2. Dispositif de shunt implantable (50 ; 50a ; 50b, 50c ; 60) selon la revendication 1, dans lequel les première et seconde ailes (58a ; 58b ; 401a ; 401b ; 401c ; 68a ; 68b) de cylindre sont conçues pour être déviées vers l'intérieur de telle sorte que des parties distales des première et seconde ailes (58a ; 58b ; 401a ; 401b ; 401c ; 68a ; 68b) de cylindre se chevauchent de manière circonférentielle.

3. Dispositif de shunt implantable (50 ; 50a ; 50b, 50c ; 60) selon la revendication 1, dans lequel des bords distaux des première et seconde ailes (58a ; 58b ; 401a ; 401b ; 401c ; 68a ; 68b) de cylindre sont incurvés l'un vers l'autre et disposés à proximité l'un de l'autre lorsque les première et seconde ailes (58a ; 58b ; 401a ; 401b ; 401c ; 68a ; 68b) de cylindre forment la forme cylindrique tubulaire (58 ; 68).

4. Dispositif de shunt implantable (50 ; 50a ; 50b, 50c ; 60) selon l'une quelconque des revendications 1 à 3, dans lequel, dans une configuration aplatie, les bras d'ancrage distal et proximal (56 ; 56a ; 56b ; 66) se projettent dans une première dimension et les première et seconde ailes (58a ; 58b ; 401a ; 401b ; 401c ; 68a ; 68b) de cylindre se projettent dans une seconde dimension qui est perpendiculaire à la première dimension.

5. Dispositif de shunt implantable (50 ; 50a ; 50b, 50c ; 60) selon l'une quelconque des revendications 1 à 4, dans lequel les ailes (58a ; 58b ; 401a ; 401b ; 401c ; 68a ; 68b) de cylindre comprennent chacune une pluralité d'entretoises latérales (417 ; 61) séparées par un ou plusieurs interstices (92) en fente latérale.

6. Dispositif de shunt implantable (50 ; 50a ; 50b, 50c ; 60) selon l'une quelconque des revendications 1 à 4, dans lequel les ailes (58a ; 58b ; 401a ; 401b ; 401c ; 68a ; 68b) de cylindre comprennent chacune une pluralité d'entretoises verticales (441 ; 541) séparées par un ou plusieurs interstices (411 ; 511) en fente verticale.

7. Dispositif de shunt implantable (50 ; 50a ; 50b, 50c ; 60) selon l'une quelconque des revendications 1 à 4, dans lequel les ailes (58a ; 58b ; 401a ; 401b ; 401c ; 68a ; 68b) de cylindre comprennent des entretoises formant une ou plusieurs rangées de cellules polygonales.

8. Dispositif de shunt implantable (50 ; 50a ; 50b, 50c ; 60) selon l'une quelconque des revendications 1 à 4, dans lequel la forme cylindrique tubulaire (58 ; 68) présente un axe qui est incliné par rapport à un plan de tissu associé au dispositif de shunt implantable (50 ; 50a ; 50b, 50c ; 60).

9. Dispositif de shunt implantable (50 ; 50a ; 50b, 50c ; 60) selon l'une quelconque des revendications 1 à 8, comprenant en outre un dispositif capteur de pression (70) fixé à l'un quelconque des bras.

10. Dispositif de shunt implantable (50 ; 50a ; 50b, 50c ; 60) selon la revendication 9, dans lequel au moins l'un des bras d'ancrage proximal et distal (56 ; 56a ; 56b ; 66) comprend un moyen (83) de retenue de capteur conçu pour fixer fermement le dispositif capteur (70) au dispositif de shunt implantable (50 ; 50a ; 50b, 50c ; 60).

11. Dispositif de shunt implantable (50 ; 50a ; 50b, 50c ; 60) selon l'une quelconque des revendications 1 à 10, dans lequel la structure d'ossature support (52 ; 62) comprend au moins une entretoise axiale.

12. Dispositif de shunt implantable (50 ; 50a ; 50b, 50c ; 60) selon la revendication 1, dans lequel les première et seconde ailes (58a ; 58b ; 401a ; 401b ; 401c ; 68a ; 68b) de cylindre sont des premier et second panneaux arqués, dans lequel, dans une configuration déployée, les premier et second panneaux arqués sont alignés radialement.

13. Dispositif de shunt implantable (50 ; 50a ; 50b, 50c ; 60) selon la revendication 12, dans lequel le contact entre des bords des premier et second panneaux arqués empêche une compression radiale de la forme cylindrique tubulaire (58 ; 68).

14. Dispositif de shunt implantable (50 ; 50a ; 50b, 50c ; 60) selon l'une quelconque des revendications 1 à 13, dans lequel les première et seconde ailes (58a ; 58b ; 401a ; 401b ; 401c ; 68a ; 68b) de cylindre sont conçues pour adopter une configuration de pose comprimée dans laquelle une première aile parmi les première et seconde ailes (58a ; 58b ; 401a ; 401b ; 401c ; 68a ; 68b) de cylindre est déviée radialement à l'intérieur d'une seconde aile parmi les première et seconde ailes de cylindre (58a ; 58b ; 401a ; 401b ; 401c ; 68a ; 68b) et la chevauche de manière circonférentielle.

15. Dispositif de shunt implantable (50 ; 50a, 50b ; 50c, 60) selon la revendication 14, dans lequel, dans la configuration de pose comprimée, la forme cylindrique tubulaire (58 ; 68) présente un diamètre qui est inférieur à un diamètre de la forme cylindrique tubulaire (58 ; 68) dans une configuration déployée dans laquelle les première et seconde ailes (58a ; 58b ; 401a ; 401b ; 401c ; 68a ; 68b) de cylindre sont alignées radialement.
